# EUROPEAN PATENT SPECIFICATION

(11) **EP 4 637 623 B1**
(45) Date of publication and mention of the grant of the patent: **18.11.2026**
(21) Application number: 23847997.6
(22) Date of filing: 18.12.2023
(51) Int. Cl.: A61C 9/00, A61B 1/00, G16H 30/40

(54) **INTRAORAL SCANNING SYSTEM**
SYSTEM FÜR INTRAORALE ABTASTUNG
SYSTÈME POUR BALAYAGE INTRA-BUCCAL

(30) Priority: 20.12.2022 US 202263434031 P; 15.12.2023 US 202318542589
(43) Date of publication of application: 29.10.2025
(73) Proprietor: Align Technology, Inc., San Jose, CA 95134 (US)
(72) Inventor: ALKABETZ, Ehud, Sde Warburg (IL); AYAL, Shai, Tel Aviv (IL); SAPHIER, Ofer, Rehovot (IL); ELBAZ, Gilad, Tel Aviv (IL); JOSHUA, Shalev, Tikva (IL); BOGRASH, Alice, Herziliya (IL); ISHAY, Eran, Tel Aviv (IL); AFRIAT, Itshak, Tel-Mond (IL)
(74) Representative: Hancox, Jonathan Christopher
(86) International application number: PCT/US2023/084645
(87) International publication number: WO 2024/137515

(56) References cited:
- US-A1- 2015 002 509
- US-A1- 2021 321 872
- US-B1- 11 423 697

## Description

### TECHNICAL FIELD

Embodiments of the present disclosure relate to the field of dentistry and, in particular, to a graphic user interface that provides viewfinder images of a region being scanned during intraoral scanning.

### BACKGROUND

In prosthodontic procedures designed to implant a dental prosthesis in the oral cavity, the dental site at which the prosthesis is to be implanted in many cases should be measured accurately and studied carefully, so that a prosthesis such as a crown, denture or bridge, for example, can be properly designed and dimensioned to fit in place. A good fit enables mechanical stresses to be properly transmitted between the prosthesis and the jaw, and to prevent infection of the gums via the interface between the prosthesis and the dental site, for example.

Some procedures also call for removable prosthetics to be fabricated to replace one or more missing teeth, such as a partial or full denture, in which case the surface contours of the areas where the teeth are missing need to be reproduced accurately so that the resulting prosthetic fits over the edentulous region with even pressure on the soft tissues.

In some practices, the dental site is prepared by a dental practitioner, and a positive physical model of the dental site is constructed using known methods. Alternatively, the dental site may be scanned to provide 3D data of the dental site. In either case, the virtual or real model of the dental site is sent to the dental lab, which manufactures the prosthesis based on the model. However, if the model is deficient or undefined in certain areas, or if the preparation was not optimally configured for receiving the prosthesis, the design of the prosthesis may be less than optimal. For example, if the insertion path implied by the preparation for a closely-fitting coping would result in the prosthesis colliding with adjacent teeth, the coping geometry has to be altered to avoid the collision, which may result in the coping design being less optimal. Further, if the area of the preparation containing a finish line lacks definition, it may not be possible to properly determine the finish line and thus the lower edge of the coping may not be properly designed. Indeed, in some circumstances, the model is rejected and the dental practitioner then re-scans the dental site, or reworks the preparation, so that a suitable prosthesis may be produced.

In orthodontic procedures it can be important to provide a model of one or both jaws. Where such orthodontic procedures are designed virtually, a virtual model of the oral cavity is also beneficial. Such a virtual model may be obtained by scanning the oral cavity directly, or by producing a physical model of the dentition, and then scanning the model with a suitable scanner.

Thus, in both prosthodontic and orthodontic procedures, obtaining a three-dimensional (3D) model of a dental site in the oral cavity is an initial procedure that is performed. When the 3D model is a virtual model, the more complete and accurate the scans of the dental site are, the higher the quality of the virtual model, and thus the greater the ability to design an optimal prosthesis or orthodontic treatment appliance(s).

US2015002509A1 discloses tools and methods for preparing digital dental models by synchronizing still images from scan video with 3D model views, enabling improved visualization and interaction, particularly for accurately identifying and marking features such as tooth margins during dental restoration design.

### SUMMARY

The invention is as defined in the appended claims.

### BRIEF DESCRIPTION OF THE DRAWINGS

Embodiments of the present disclosure are illustrated by way of example, and not by way of limitation, in the figures of the accompanying drawings.
**FIG.** 1 illustrates one embodiment of a system for performing intraoral scanning and/or generating a virtual three-dimensional model of an dental site.
**FIG. 2A** is a schematic illustration of a handheld intraoral scanner with a plurality cameras disposed within a probe at a distal end of the intraoral scanner, in accordance with some applications of the present disclosure.
**FIGS. 2B-2C** comprise schematic illustrations of positioning configurations for cameras and structured light projectors of an intraoral scanner, in accordance with some applications of the present disclosure.
**Fig. 2D** is a chart depicting a plurality of different configurations for the position of structured light projectors and cameras in a probe of an intraoral scanner, in accordance with some applications of the present disclosure.
**FIG. 3A** illustrates 2D images generated by an array of cameras of an intraoral scanner, in accordance with embodiments of the present disclosure.
**FIG. 3B** illustrates 2D images generated by an array of cameras of an intraoral scanner, in accordance with embodiments of the present disclosure.
**FIG. 3C** illustrates 2D images generated by an array of cameras of an intraoral scanner, in accordance with embodiments of the present disclosure.
**FIG. 3D** illustrates a view of a graphical user interface of an intraoral scan application that includes a 3D surface and a selected 2D image of a current field of view of an intraoral scanner, in accordance with embodiments of the present disclosure.
**FIG. 3E** illustrates a view of a graphical user interface of an intraoral scan application that includes a 3D surface and a selected 2D image of a current field of view of an intraoral scanner, in accordance with embodiments of the present disclosure.
**FIG. 4** illustrates reference frames of multiple cameras of an intraoral scanner, in accordance with an embodiment of the present disclosure.
**FIG. 5** illustrates a flow chart of an embodiment for a method of automatically selecting an intraoral image to display from a set of intraoral images, in accordance with embodiments of the present disclosure.
**FIG. 6** illustrates a flow chart of an embodiment for a method of recommending an intraoral image to display from a set of intraoral images, in accordance with embodiments of the present disclosure.
**FIG. 7** illustrates a flow chart of an embodiment for a method of automatically selecting multiple intraoral images to display from a set of intraoral images and generating a combined image from the selected images, in accordance with embodiments of the present disclosure.
**FIG. 8** illustrates a flow chart of an embodiment for a method of determining which image from a set of images to select for display using a trained machine learning model, in accordance with embodiments of the present disclosure.
**FIG. 9** illustrates a flow chart of an embodiment for a method of determining which image from a set of images to select for display, in accordance with embodiments of the present disclosure.
**FIG. 10** illustrates a flow chart of an embodiment for a method of automatically selecting an intraoral image to display from a set of intraoral images, taking into account selections from prior sets of intraoral images, in accordance with embodiments of the present disclosure.
**FIG. 11** illustrates a block diagram of an example computing device, in accordance with embodiments of the present disclosure.

### DETAILED DESCRIPTION

Described herein are methods and systems for simplifying the process of performing intraoral scanning and for providing useful real time visualizations of intraoral objects (e.g., dental sites) associated with the intraoral scanning process during intraoral scanning. In particular, embodiments described herein include systems and methods for selecting images to output to a display during intraoral scanning to, for example, enable a doctor or technician to understand the current region of a mouth being scanned. In embodiments, an intraoral scan application can continuously adjust selection of one or more cameras during intraoral scanning, where each camera may generate images that provide different views of a 3D surface being scanned.

In embodiments, an intraoral scanner may include multiple cameras (e.g., an array of cameras), each of which may have a different position and/or orientation on the intraoral scanner, and each of which may provide a different point of view of a surface being scanned. Each of the cameras may periodically generate intraoral images (also referred to herein simply as images). A set of images may be generated, where the set may include an image generated by each of the cameras. Processing logic may perform one or more operations on a received set of images to select which of the images to output to a display, and/or which camera to select. The selected image may be the image that provides the best or most useful/helpful information to a user of the intraoral scanner.

For intraoral scanners that include multiple cameras, displaying images from each of the images may be confusing to a user. The user may not be able to easily understand how the scanner is positioned in a patient's oral cavity from the multiple images. By implementing embodiments set forth herein, an image that provides most useful information may be selected and output to a user to enable that user to easily and intuitively determine what is being scanned and where/how the scanner is positioned in a patient's mouth.

Various embodiments are described herein. It should be understood that these various embodiments may be implemented as stand-alone solutions and/or may be combined. Accordingly, references to an embodiment, or one embodiment, may refer to the same embodiment and/or to different embodiments. Some embodiments are discussed herein with reference to intraoral scans and intraoral images. However, it should be understood that embodiments described with reference to intraoral scans also apply to lab scans or model/impression scans. A lab scan or model/impression scan may include one or more images of a dental site or of a model or impression of a dental site.

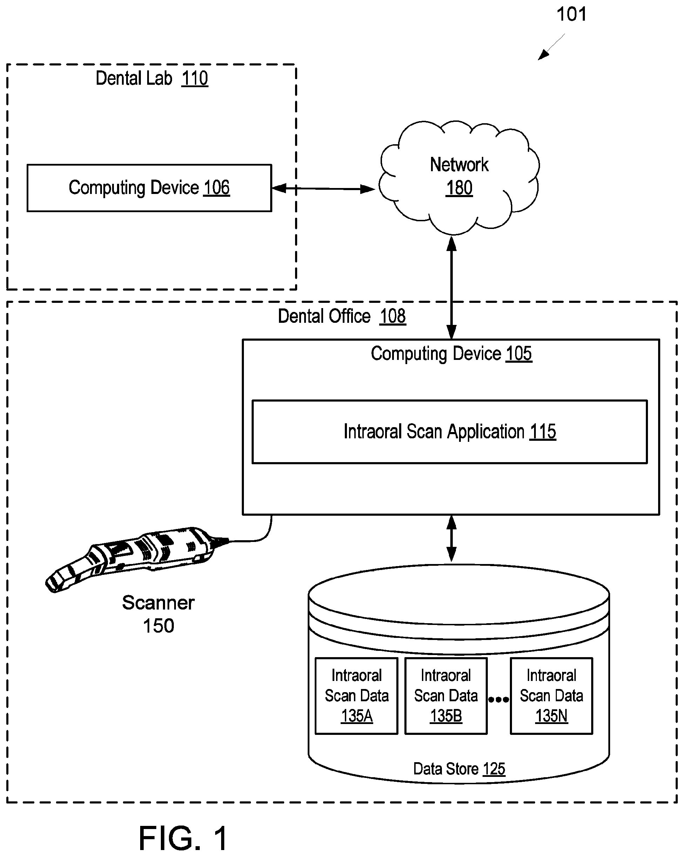

**FIG. 1** illustrates one embodiment of a system 101 for performing intraoral scanning and/or generating a three-dimensional (3D) surface and/or a virtual three-dimensional model of a dental site. System 101 includes a dental office 108 and optionally one or more dental lab 110. The dental office 108 and the dental lab 110 each include a computing device 105, 106, where the computing devices 105, 106 may be connected to one another via a network 180. The network 180 may be a local area network (LAN), a public wide area network (WAN) (e.g., the Internet), a private WAN (e.g., an intranet), or a combination thereof.

Computing device 105 may be coupled to one or more intraoral scanner 150 (also referred to as a scanner) and/or a data store 125 via a wired or wireless connection. In one embodiment, multiple scanners 150 in dental office 108 wirelessly connect to computing device 105. In one embodiment, scanner 150 is wirelessly connected to computing device 105 via a direct wireless connection. In one embodiment, scanner 150 is wirelessly connected to computing device 105 via a wireless network. In one embodiment, the wireless network is a Wi-Fi network. In one embodiment, the wireless network is a Bluetooth network, a Zigbee network, or some other wireless network. In one embodiment, the wireless network is a wireless mesh network, examples of which include a Wi-Fi mesh network, a Zigbee mesh network, and so on. In an example, computing device 105 may be physically connected to one or more wireless access points and/or wireless routers (e.g., Wi-Fi access points/routers). Intraoral scanner 150 may include a wireless module such as a Wi-Fi module, and via the wireless module may join the wireless network via the wireless access point/router.

Computing device 106 may also be connected to a data store (not shown). The data stores may be local data stores and/or remote data stores. Computing device 105 and computing device 106 may each include one or more processing devices, memory, secondary storage, one or more input devices (e.g., such as a keyboard, mouse, tablet, touchscreen, microphone, camera, and so on), one or more output devices (e.g., a display, printer, touchscreen, speakers, etc.), and/or other hardware components.

In embodiments, scanner 150 includes an inertial measurement unit (IMU). The IMU may include an accelerometer, a gyroscope, a magnetometer, a pressure sensor and/or other sensor. For example, scanner 150 may include one or more micro-electromechanical system (MEMS) IMU. The IMU may generate inertial measurement data (also referred to as movement data), including acceleration data, rotation data, and so on.

Computing device 105 and/or data store 125 may be located at dental office 108 (as shown), at dental lab 110, or at one or more other locations such as a server farm that provides a cloud computing service. Computing device 105 and/or data store 125 may connect to components that are at a same or a different location from computing device 105 (e.g., components at a second location that is remote from the dental office 108, such as a server farm that provides a cloud computing service). For example, computing device 105 may be connected to a remote server, where some operations of intraoral scan application 115 are performed on computing device 105 and some operations of intraoral scan application 115 are performed on the remote server.

Some additional computing devices may be physically connected to the computing device 105 via a wired connection. Some additional computing devices may be wirelessly connected to computing device 105 via a wireless connection, which may be a direct wireless connection or a wireless connection via a wireless network. In embodiments, one or more additional computing devices may be mobile computing devices such as laptops, notebook computers, tablet computers, mobile phones, portable game consoles, and so on. In embodiments, one or more additional computing devices may be traditionally stationary computing devices, such as desktop computers, set top boxes, game consoles, and so on. The additional computing devices may act as thin clients to the computing device 105. In one embodiment, the additional computing devices access computing device 105 using remote desktop protocol (RDP). In one embodiment, the additional computing devices access computing device 105 using virtual network control (VNC). Some additional computing devices may be passive clients that do not have control over computing device 105 and that receive a visualization of a user interface of intraoral scan application 115. In one embodiment, one or more additional computing devices may operate in a master mode and computing device 105 may operate in a slave mode.

Intraoral scanner 150 may include a probe (e.g., a hand held probe) for optically capturing three-dimensional structures. The intraoral scanner 150 may be used to perform an intraoral scan of a patient's oral cavity. An intraoral scan application 115 running on computing device 105 may communicate with the scanner 150 to effectuate the intraoral scan. A result of the intraoral scan may be intraoral scan data 135A, 135B through 135N that may include one or more sets of intraoral scans and/or sets of intraoral 2D images. Each intraoral scan may include a 3D image or point cloud that may include depth information (e.g., a height map) of a portion of a dental site. In embodiments, intraoral scans include x, y and z information.

Intraoral scan data 135A-N may also include color 2D images and/or images of particular wavelengths (e.g., near-infrared (NIR) images, infrared images, ultraviolet images, etc.) of a dental site in embodiments. In embodiments, intraoral scanner 150 alternates between generation of 3D intraoral scans and one or more types of 2D intraoral images (e.g., color images, NIR images, etc.) during scanning. For example, one or more 2D color images may be generated between generation of a fourth and fifth intraoral scan by outputting white light and capturing reflections of the white light using multiple cameras.

Intraoral scanner 150 may include multiple different cameras (e.g., each of which may include one or more image sensors) that generate intraoral images (e.g., 2D color images) of different regions of a patient's dental arch concurrently. These intraoral image (e.g., 2D images) may be assessed, and one or more of the images and/or the cameras that generated the images may be selected for output to a display. If multiple images/cameras are selected, the multiple images may be stitched together to form a single 2D image representation of a larger field of view that includes a combination of the fields of view of the multiple cameras that were selected. Intraoral 2D images may include 2D color images, 2D infrared or near-infrared (NIRI) images, and/or 2D images generated under other specific lighting conditions (e.g., 2D ultraviolet images). The 2D images may be used by a user of the intraoral scanner to determine where the scanning face of the intraoral scanner is directed and/or to determine other information about a dental site being scanned.

The scanner 150 may transmit the intraoral scan data 135A, 135B through 135N to the computing device 105. Computing device 105 may store the intraoral scan data 135A-135N in data store 125.

According to an example, a user (e.g., a practitioner) may subject a patient to intraoral scanning. In doing so, the user may apply scanner 150 to one or more patient intraoral locations. The scanning may be divided into one or more segments (also referred to as roles). As an example, the segments may include a lower dental arch of the patient, an upper dental arch of the patient, one or more preparation teeth of the patient (e.g., teeth of the patient to which a dental device such as a crown or other dental prosthetic will be applied), one or more teeth which are contacts of preparation teeth (e.g., teeth not themselves subject to a dental device but which are located next to one or more such teeth or which interface with one or more such teeth upon mouth closure), and/or patient bite (e.g., scanning performed with closure of the patient's mouth with the scan being directed towards an interface area of the patient's upper and lower teeth). Via such scanner application, the scanner 150 may provide intraoral scan data 135A-N to computing device 105. The intraoral scan data 135A-N may be provided in the form of intraoral scan data sets, each of which may include 2D intraoral images (e.g., color 2D images) and/or 3D intraoral scans of particular teeth and/or regions of an dental site. In one embodiment, separate intraoral scan data sets are created for the maxillary arch, for the mandibular arch, for a patient bite, and/or for each preparation tooth. Alternatively, a single large intraoral scan data set is generated (e.g., for a mandibular and/or maxillary arch). Intraoral scans may be provided from the scanner 150 to the computing device 105 in the form of one or more points (e.g., one or more pixels and/or groups of pixels). For instance, the scanner 150 may provide an intraoral scan as one or more point clouds. The intraoral scans may each comprise height information (e.g., a height map that indicates a depth for each pixel).

The manner in which the oral cavity of a patient is to be scanned may depend on the procedure to be applied thereto. For example, if an upper or lower denture is to be created, then a full scan of the mandibular or maxillary edentulous arches may be performed. In contrast, if a bridge is to be created, then just a portion of a total arch may be scanned which includes an edentulous region, the neighboring preparation teeth (e.g., abutment teeth) and the opposing arch and dentition. Alternatively, full scans of upper and/or lower dental arches may be performed if a bridge is to be created.

By way of non-limiting example, dental procedures may be broadly divided into prosthodontic (restorative) and orthodontic procedures, and then further subdivided into specific forms of these procedures. Additionally, dental procedures may include identification and treatment of gum disease, sleep apnea, and intraoral conditions. The term prosthodontic procedure refers, inter alia, to any procedure involving the oral cavity and directed to the design, manufacture or installation of a dental prosthesis at a dental site within the oral cavity (dental site), or a real or virtual model thereof, or directed to the design and preparation of the dental site to receive such a prosthesis. A prosthesis may include any restoration such as crowns, veneers, inlays, onlays, implants and bridges, for example, and any other artificial partial or complete denture. The term orthodontic procedure refers, inter alia, to any procedure involving the oral cavity and directed to the design, manufacture or installation of orthodontic elements at a dental site within the oral cavity, or a real or virtual model thereof, or directed to the design and preparation of the dental site to receive such orthodontic elements. These elements may be appliances including but not limited to brackets and wires, retainers, clear aligners, or functional appliances.

In embodiments, intraoral scanning may be performed on a patient's oral cavity during a visitation of dental office 108. The intraoral scanning may be performed, for example, as part of a semi-annual or annual dental health checkup. The intraoral scanning may also be performed before, during and/or after one or more dental treatments, such as orthodontic treatment and/or prosthodontic treatment. The intraoral scanning may be a full or partial scan of the upper and/or lower dental arches, and may be performed in order to gather information for performing dental diagnostics, to generate a treatment plan, to determine progress of a treatment plan, and/or for other purposes. The dental information (intraoral scan data 135A-N) generated from the intraoral scanning may include 3D scan data, 2D color images, NIRI and/or infrared images, and/or ultraviolet images, of all or a portion of the upper jaw and/or lower jaw. The intraoral scan data 135A-N may further include one or more intraoral scans showing a relationship of the upper dental arch to the lower dental arch (e.g., showing a bite). These intraoral scans may be usable to determine a patient bite and/or to determine occlusal contact information for the patient. The patient bite may include determined relationships between teeth in the upper dental arch and teeth in the lower dental arch.

For many prosthodontic procedures (e.g., to create a crown, bridge, veneer, etc.), an existing tooth of a patient is ground down to a stump. The ground tooth is referred to herein as a preparation tooth, or simply a preparation. The preparation tooth has a margin line (also referred to as a finish line), which is a border between a natural (unground) portion of the preparation tooth and the prepared (ground) portion of the preparation tooth. The preparation tooth is typically created so that a crown or other prosthesis can be mounted or seated on the preparation tooth. In many instances, the margin line of the preparation tooth is sub-gingival (below the gum line).

Intraoral scanners may work by moving the scanner 150 inside a patient's mouth to capture all viewpoints of one or more tooth. During scanning, the scanner 150 is calculating distances to solid surfaces in some embodiments. These distances may be recorded as images called 'height maps' or as point clouds in some embodiments. Each scan (e.g., optionally height map or point cloud) is overlapped algorithmically, or 'stitched', with the previous set of scans to generate a growing 3D surface. As such, each scan is associated with a rotation in space, or a projection, to how it fits into the 3D surface.

During intraoral scanning, the intraoral scanner 150 periodically or continuously generates sets of intraoral images (e.g., 2D intraoral images), where each image in a set of intraoral images is generated by a different camera of the intraoral scanner 150. Intraoral scan application 115 processes received sets of intraoral images to determine which camera to select and/or which image to output to a display for the sets of intraoral images. Different cameras may be selected for different sets of intraoral images. For example, at a first time during an intraoral scanning session a first camera may be selected, and images generated by that first camera are output to a display (e.g., to show a viewfinder image of the intraoral scanner). Later during the intraoral scanning session, after the intraoral scanner has been moved within a patient's mouth, a second camera may be selected, and images generated by that second camera are output to the display. The selected camera may be a camera that, for a current position/orientation of the scanner 150, generates images that contain most useful information.

During intraoral scanning, intraoral scan application 115 may register and stitch together two or more intraoral scans generated thus far from the intraoral scan session to generate a growing 3D surface. In one embodiment, performing registration includes capturing 3D data of various points of a surface in multiple scans, and registering the scans by computing transformations between the scans. One or more 3D surfaces may be generated based on the registered and stitched together intraoral scans during the intraoral scanning. The one or more 3D surfaces may be output to a display so that a doctor or technician can view their scan progress thus far. As each new intraoral scan is captured and registered to previous intraoral scans and/or a 3D surface, the one or more 3D surfaces may be updated, and the updated 3D surface(s) may be output to the display. A view of the 3D surface(s) may be periodically or continuously updated according to one or more viewing modes of the intraoral scan application. In one viewing mode, the 3D surface may be continuously updated such that an orientation of the 3D surface that is displayed aligns with a field of view of the intraoral scanner (e.g., so that a portion of the 3D surface that is based on a most recently generated intraoral scan is approximately centered on the display or on a window of the display) and a user sees what the intraoral scanner sees. In one viewing mode, a position and orientation of the 3D surface is static, and an image of the intraoral scanner is optionally shown to move relative to the stationary 3D surface. Other viewing modes may include zoomed in viewing modes that show magnified views of one or more regions of the 3D surface (e.g., of intraoral areas of interest (AOls)). Other viewing modes are also possible.

In embodiments, separate 3D surfaces are generated for the upper jaw and the lower jaw. This process may be performed in real time or near-real time to provide an updated view of the captured 3D surfaces during the intraoral scanning process.

When a scan session or a portion of a scan session associated with a particular scanning role (e.g., upper jaw role, lower jaw role, bite role, etc.) is complete (e.g., all scans for an dental site or dental site have been captured), intraoral scan application 115 may generate a virtual 3D model of one or more scanned dental sites (e.g., of an upper jaw and a lower jaw). The final 3D model may be a set of 3D points and their connections with each other (i.e. a mesh). To generate the virtual 3D model, intraoral scan application 115 may register and stitch together the intraoral scans generated from the intraoral scan session that are associated with a particular scanning role. The registration performed at this stage may be more accurate than the registration performed during the capturing of the intraoral scans, and may take more time to complete than the registration performed during the capturing of the intraoral scans. In one embodiment, performing scan registration includes capturing 3D data of various points of a surface in multiple scans, and registering the scans by computing transformations between the scans. The 3D data may be projected into a 3D space of a 3D model to form a portion of the 3D model. The intraoral scans may be integrated into a common reference frame by applying appropriate transformations to points of each registered scan and projecting each scan into the 3D space.

In one embodiment, registration is performed for adjacent or overlapping intraoral scans (e.g., each successive frame of an intraoral video). Registration algorithms are carried out to register two adjacent or overlapping intraoral scans and/or to register an intraoral scan with a 3D model, which essentially involves determination of the transformations which align one scan with the other scan and/or with the 3D model. Registration may involve identifying multiple points in each scan (e.g., point clouds) of a scan pair (or of a scan and the 3D model), surface fitting to the points, and using local searches around points to match points of the two scans (or of the scan and the 3D model). For example, intraoral scan application 115 may match points of one scan with the closest points interpolated on the surface of another scan, and iteratively minimize the distance between matched points. Other registration techniques may also be used.

Intraoral scan application 115 may repeat registration for all intraoral scans of a sequence of intraoral scans to obtain transformations for each intraoral scan, to register each intraoral scan with previous intraoral scan(s) and/or with a common reference frame (e.g., with the 3D model). Intraoral scan application 115 may integrate intraoral scans into a single virtual 3D model by applying the appropriate determined transformations to each of the intraoral scans. Each transformation may include rotations about one to three axes and translations within one to three planes.

Intraoral scan application 115 may generate one or more 3D models from intraoral scans, and may display the 3D models to a user (e.g., a doctor) via a graphical user interface (GUI). The 3D models can then be checked visually by the doctor. The doctor can virtually manipulate the 3D models via the user interface with respect to up to six degrees of freedom (i.e., translated and/or rotated with respect to one or more of three mutually orthogonal axes) using suitable user controls (hardware and/or virtual) to enable viewing of the 3D model from any desired direction. In some embodiments, a trajectory of a virtual camera imaging the 3D model is automatically computed, and the 3D model is shown according to the determined trajectory. Accordingly, the doctor may review (e.g., visually inspect) the generated 3D model of a dental site and determine whether the 3D model is acceptable (e.g., whether a margin line of a preparation tooth is accurately represented in the 3D model) without manually controlling or manipulating a view of the 3D model. For example, in some embodiments, the intraoral scan application 115 automatically generates a sequence of views of the 3D model and cycles through the views in the generated sequence. This may include zooming in, zooming out, panning, rotating, and so on.

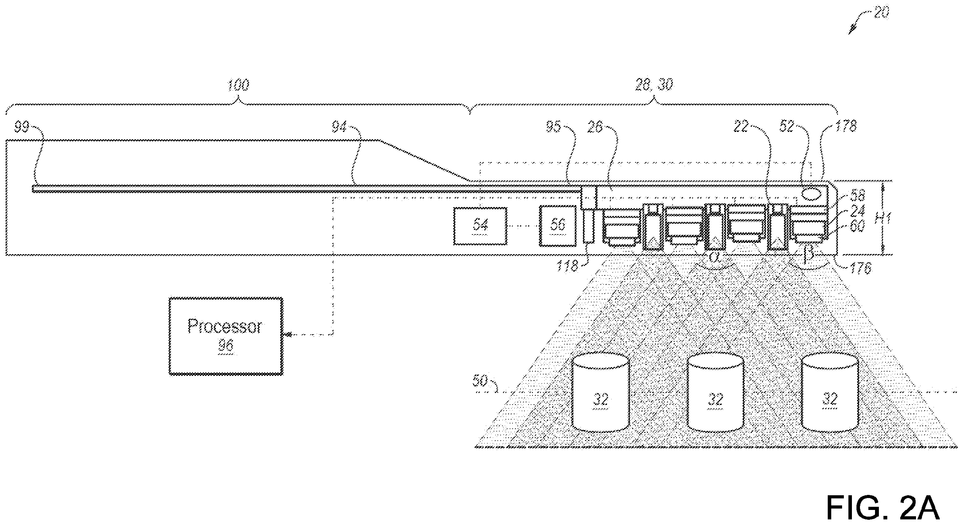

Reference is now made to **FIG. 2A****,** which is a schematic illustration of an intraoral scanner 20 comprising an elongate handheld wand, in accordance with some applications of the present disclosure. The intraoral scanner 20 may correspond to intraoral scanner 150 of **FIG. 1** in embodiments. Intraoral scanner 20 includes a plurality of structured light projectors 22 and a plurality of cameras 24 that are coupled to a rigid structure 26 disposed within a probe 28 at a distal end 30 of the intraoral scanner 20. In some applications, during an intraoral scanning procedure, probe 28 is inserted into the oral cavity of a subject or patient.

For some applications, structured light projectors 22 are positioned within probe 28 such that each structured light projector 22 faces an object 32 outside of intraoral scanner 20 that is placed in its field of illumination, as opposed to positioning the structured light projectors in a proximal end of the handheld wand and illuminating the object by reflection of light off a mirror and subsequently onto the object. Alternatively, the structured light projectors may be disposed at a proximal end of the handheld wand. Similarly, for some applications, cameras 24 are positioned within probe 28 such that each camera 24 faces an object 32 outside of intraoral scanner 20 that is placed in its field of view, as opposed to positioning the cameras in a proximal end of the intraoral scanner and viewing the object by reflection of light off a mirror and into the camera. This positioning of the projectors and the cameras within probe 28 enables the scanner to have an overall large field of view while maintaining a low profile probe. Alternatively, the cameras may be disposed in a proximal end of the handheld wand.

In some applications, cameras 24 each have a large field of view β (beta) of at least 45 degrees, e.g., at least 70 degrees, e.g., at least 80 degrees, e.g., 85 degrees. In some applications, the field of view may be less than 120 degrees, e.g., less than 100 degrees, e.g., less than 90 degrees. In one embodiment, a field of view β (beta) for each camera is between 80 and 90 degrees, which may be particularly useful because it provided a good balance among pixel size, field of view and camera overlap, optical quality, and cost. Cameras 24 may include an image sensor 58 and objective optics 60 including one or more lenses. To enable close focus imaging, cameras 24 may focus at an object focal plane 50 that is located between 1 mm and 30 mm, e.g., between 4 mm and 24 mm, e.g., between 5 mm and 11 mm, e.g., 9 mm - 10 mm, from the lens that is farthest from the sensor. In some applications, cameras 24 may capture images at a frame rate of at least 30 frames per second, e.g., at a frame of at least 75 frames per second, e.g., at least 100 frames per second. In some applications, the frame rate may be less than 200 frames per second.

A large field of view achieved by combining the respective fields of view of all the cameras may improve accuracy due to reduced amount of image stitching errors, especially in edentulous regions, where the gum surface is smooth and there may be fewer clear high resolution 3D features. Having a larger field of view enables large smooth features, such as the overall curve of the tooth, to appear in each image frame, which improves the accuracy of stitching respective surfaces obtained from multiple such image frames.

Similarly, structured light projectors 22 may each have a large field of illumination α (alpha) of at least 45 degrees, e.g., at least 70 degrees. In some applications, field of illumination α (alpha) may be less than 120 degrees, e.g., than 100 degrees.

For some applications, in order to improve image capture, each camera 24 has a plurality of discrete preset focus positions, in each focus position the camera focusing at a respective object focal plane 50. Each of cameras 24 may include an autofocus actuator that selects a focus position from the discrete preset focus positions in order to improve a given image capture. Additionally or alternatively, each camera 24 includes an optical aperture phase mask that extends a depth of focus of the camera, such that images formed by each camera are maintained focused over all object distances located between 1 mm and 30 mm, e.g., between 4 mm and 24 mm, e.g., between 5 mm and 11 mm, e.g., 9 mm - 10 mm, from the lens that is farthest from the sensor.

In some applications, structured light projectors 22 and cameras 24 are coupled to rigid structure 26 in a closely packed and/or alternating fashion, such that (a) a substantial part of each camera's field of view overlaps the field of view of neighboring cameras, and (b) a substantial part of each camera's field of view overlaps the field of illumination of neighboring projectors. Optionally, at least 20%, e.g., at least 50%, e.g., at least 75% of the projected pattern of light are in the field of view of at least one of the cameras at an object focal plane 50 that is located at least 4 mm from the lens that is farthest from the sensor. Due to different possible configurations of the projectors and cameras, some of the projected pattern may never be seen in the field of view of any of the cameras, and some of the projected pattern may be blocked from view by object 32 as the scanner is moved around during a scan.

Rigid structure 26 may be a non-flexible structure to which structured light projectors 22 and cameras 24 are coupled so as to provide structural stability to the optics within probe 28. Coupling all the projectors and all the cameras to a common rigid structure helps maintain geometric integrity of the optics of each structured light projector 22 and each camera 24 under varying ambient conditions, e.g., under mechanical stress as may be induced by the subject's mouth. Additionally, rigid structure 26 helps maintain stable structural integrity and positioning of structured light projectors 22 and cameras 24 with respect to each other.

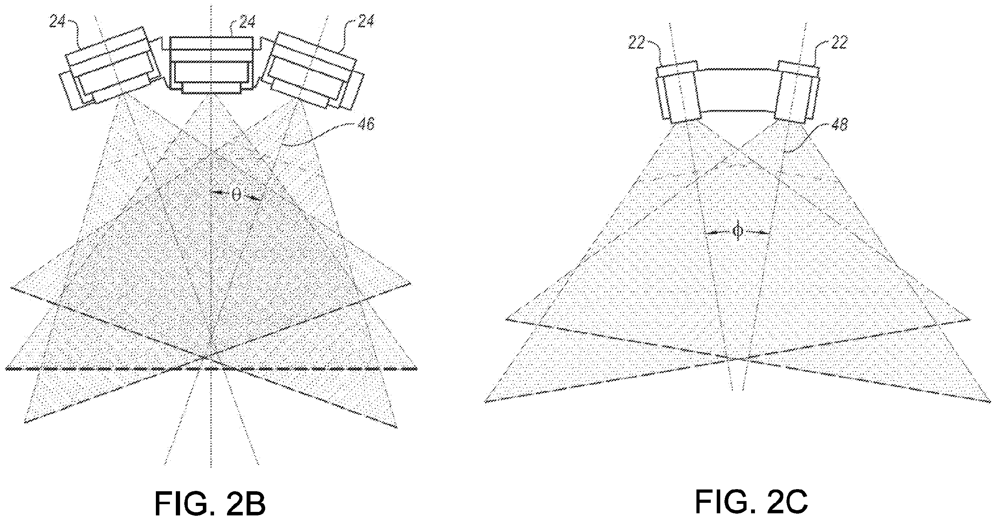

Reference is now made to **FIGS. 2B-2C****,** which include schematic illustrations of a positioning configuration for cameras 24 and structured light projectors 22 respectively, in accordance with some applications of the present disclosure. For some applications, in order to improve the overall field of view and field of illumination of the intraoral scanner 20, cameras 24 and structured light projectors 22 are positioned such that they do not all face the same direction. For some applications, such as is shown in **FIG. 2B****,** a plurality of cameras 24 are coupled to rigid structure 26 such that an angle θ (theta) between two respective optical axes 46 of at least two cameras 24 is 90 degrees or less, e.g., 35 degrees or less. Similarly, for some applications, such as is shown in **FIG. 2C****,** a plurality of structured light projectors 22 are coupled to rigid structure 26 such that an angle φ (phi) between two respective optical axes 48 of at least two structured light projectors 22 is 90 degrees or less, e.g., 35 degrees or less.

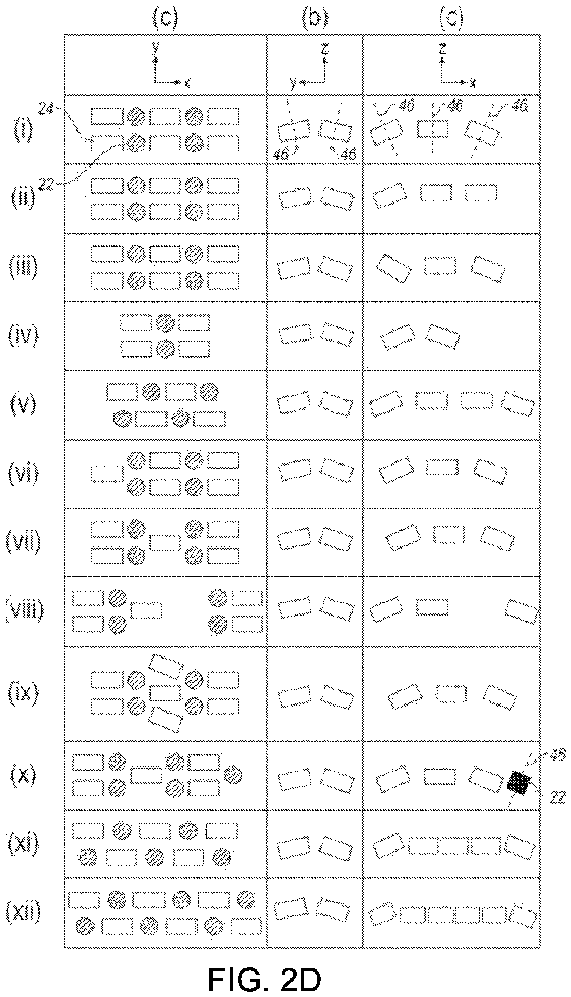

Reference is now made to **FIG. 2D****,** which is a chart depicting a plurality of different configurations for the position of structured light projectors 22 and cameras 24 in probe 28, in accordance with some applications of the present disclosure. Structured light projectors 22 are represented in **FIG. 2D** by circles and cameras 24 are represented in **FIG. 2D** by rectangles. It is noted that rectangles are used to represent the cameras, since typically, each image sensor 58 and the field of view β (beta) of each camera 24 have aspect ratios of 1:2. Column (a) of **FIG. 2D** shows a bird's eye view of the various configurations of structured light projectors 22 and cameras 24. The x-axis as labeled in the first row of column (a) corresponds to a central longitudinal axis of probe 28. Column (b) shows a side view of cameras 24 from the various configurations as viewed from a line of sight that is coaxial with the central longitudinal axis of probe 28 and substantially parallel to a viewing axis of the intraoral scanner. Similarly to as shown in **FIG. 2B****,** column (b) of **Fig. 2D** shows cameras 24 positioned so as to have optical axes 46 at an angle of 90 degrees or less, e.g., 35 degrees or less, with respect to each other. Column (c) shows a side view of cameras 24 of the various configurations as viewed from a line of sight that is perpendicular to the central longitudinal axis of probe 28.

Typically, the distal-most (toward the positive x-direction in **FIG. 2D****)** and proximal-most (toward the negative x-direction in **FIG. 2D****)** cameras 24 are positioned such that their optical axes 46 are slightly turned inwards, e.g., at an angle of 90 degrees or less, e.g., 35 degrees or less, with respect to the next closest camera 24. The camera(s) 24 that are more centrally positioned, i.e., not the distal-most camera 24 nor proximal-most camera 24, are positioned so as to face directly out of the probe, their optical axes 46 being substantially perpendicular to the central longitudinal axis of probe 28. It is noted that in row (xi) a projector 22 is positioned in the distal-most position of probe 28, and as such the optical axis 48 of that projector 22 points inwards, allowing a larger number of spots 33 projected from that particular projector 22 to be seen by more cameras 24.

In embodiments, the number of structured light projectors 22 in probe 28 may range from two, e.g., as shown in row (iv) of **FIG. 2D****,** to six, e.g., as shown in row (xii). Typically, the number of cameras 24 in probe 28 may range from four, e.g., as shown in rows (iv) and (v), to seven, e.g., as shown in row (ix). It is noted that the various configurations shown in **FIG. 2D** are by way of example and not limitation, and that the scope of the present disclosure includes additional configurations not shown. For example, the scope of the present disclosure includes fewer or more than five projectors 22 positioned in probe 28 and fewer or more than seven cameras positioned in probe 28.

In an example application, an apparatus for intraoral scanning (e.g., an intraoral scanner 150) includes an elongate handheld wand comprising a probe at a distal end of the elongate handheld wand, at least two light projectors disposed within the probe, and at least four cameras disposed within the probe. Each light projector may include at least one light source configured to generate light when activated, and a pattern generating optical element that is configured to generate a pattern of light when the light is transmitted through the pattern generating optical element. Each of the at least four cameras may include a camera sensor (also referred to as an image sensor) and one or more lenses, wherein each of the at least four cameras is configured to capture a plurality of images that depict at least a portion of the projected pattern of light on an intraoral surface. A majority of the at least two light projectors and the at least four cameras may be arranged in at least two rows that are each approximately parallel to a longitudinal axis of the probe, the at least two rows comprising at least a first row and a second row.

In a further application, a distal-most camera along the longitudinal axis and a proximal-most camera along the longitudinal axis of the at least four cameras are positioned such that their optical axes are at an angle of 90 degrees or less with respect to each other from a line of sight that is perpendicular to the longitudinal axis. Cameras in the first row and cameras in the second row may be positioned such that optical axes of the cameras in the first row are at an angle of 90 degrees or less with respect to optical axes of the cameras in the second row from a line of sight that is coaxial with the longitudinal axis of the probe. A remainder of the at least four cameras other than the distal-most camera and the proximal-most camera have optical axes that are substantially parallel to the longitudinal axis of the probe. Each of the at least two rows may include an alternating sequence of light projectors and cameras.

In a further application, the at least four cameras comprise at least five cameras, the at least two light projectors comprise at least five light projectors, a proximal-most component in the first row is a light projector, and a proximal-most component in the second row is a camera.

In a further application, the distal-most camera along the longitudinal axis and the proximal-most camera along the longitudinal axis are positioned such that their optical axes are at an angle of 35 degrees or less with respect to each other from the line of sight that is perpendicular to the longitudinal axis. The cameras in the first row and the cameras in the second row may be positioned such that the optical axes of the cameras in the first row are at an angle of 35 degrees or less with respect to the optical axes of the cameras in the second row from the line of sight that is coaxial with the longitudinal axis of the probe.

In a further application, the at least four cameras may have a combined field of view of 25-45 mm along the longitudinal axis and a field of view of 20-40 mm along a z-axis corresponding to distance from the probe.

Returning to FIG. 2A, for some applications, there is at least one uniform light projector 118 (which may be an unstructured light projector that projects light across a range of wavelengths) coupled to rigid structure 26. Uniform light projector 118 may transmit white light onto object 32 being scanned. At least one camera, e.g., one of cameras 24, captures two-dimensional color images (e.g., color intraoral images) of object 32 using illumination from uniform light projector 118.

Processor 96 may run a surface reconstruction algorithm that may use detected patterns (e.g., dot patterns) projected onto object 32 to generate a 3D surface of the object 32. In some embodiments, the processor 96 may combine at least one 3D scan captured using illumination from structured light projectors 22 with a plurality of intraoral 2D images captured using illumination from uniform light projector 118 in order to generate a digital three-dimensional image of the intraoral three-dimensional surface. Using a combination of structured light and uniform illumination enhances the overall capture of the intraoral scanner and may help reduce the number of options that processor 96 needs to consider when running a correspondence algorithm used to detect depth values for object 32. In one embodiment, the intraoral scanner and correspondence algorithm described in U.S. Application No. 16/446,181, filed June 19, 2019, is used. U.S. Application No. 16/446,181, filed June 19, 2019. In embodiments, processor 92 may be a processor of computing device 105 of **FIG. 1****.** Alternatively, processor 92 may be a processor integrated into the intraoral scanner 20.

For some applications, all data points taken at a specific time are used as a rigid point cloud, and multiple such point clouds are captured at a frame rate of over 10 captures per second. The plurality of point clouds are then stitched together using a registration algorithm, e.g., iterative closest point (ICP), to create a dense point cloud. A surface reconstruction algorithm may then be used to generate a representation of the surface of object 32.

For some applications, at least one temperature sensor 52 is coupled to rigid structure 26 and measures a temperature of rigid structure 26. Temperature control circuitry 54 disposed within intraoral scanner 20 (a) receives data from temperature sensor 52 indicative of the temperature of rigid structure 26 and (b) activates a temperature control unit 56 in response to the received data. Temperature control unit 56, e.g., a PID controller, keeps probe 28 at a desired temperature (e.g., between 35 and 43 degrees Celsius, between 37 and 41 degrees Celsius, etc.). Keeping probe 28 above 35 degrees Celsius, e.g., above 37 degrees Celsius, reduces fogging of the glass surface of intraoral scanner 20, through which structured light projectors 22 project and cameras 24 view, as probe 28 enters the intraoral cavity, which is typically around or above 37 degrees Celsius. Keeping probe 28 below 43 degrees, e.g., below 41 degrees Celsius, prevents discomfort or pain.

In some embodiments, heat may be drawn out of the probe 28 via a heat conducting element 94, e.g., a heat pipe, that is disposed within intraoral scanner 20, such that a distal end 95 of heat conducting element 94 is in contact with rigid structure 26 and a proximal end 99 is in contact with a proximal end 100 of intraoral scanner 20. Heat is thereby transferred from rigid structure 26 to proximal end 100 of intraoral scanner 20. Alternatively or additionally, a fan disposed in a handle region 174 of intraoral scanner 20 may be used to draw heat out of probe 28.

**FIGS. 2A-2D** illustrate one type of intraoral scanner that can be used for embodiments of the present disclosure. However, it should be understood that embodiments are not limited to the illustrated type of intraoral scanner. In one embodiment, intraoral scanner 150 corresponds to the intraoral scanner described in U.S. Application No. 16/910,042, filed June 23, 2020 and entitled "Intraoral 3D Scanner Employing Multiple Miniature Cameras and Multiple Miniature Pattern Projectors". In one embodiment, intraoral scanner 150 corresponds to the intraoral scanner described in U.S. Application No. 16/446,181, filed June 19, 2019 and entitled "Intraoral 3D Scanner Employing Multiple Miniature Cameras and Multiple Miniature Pattern Projectors".

In some embodiments an intraoral scanner that performs confocal focusing to determine depth information may be used. Such an intraoral scanner may include a light source and/or illumination module that emits light (e.g., a focused light beam or array of focused light beams). The light passes through a polarizer and through a unidirectional mirror or beam splitter (e.g., a polarizing beam splitter) that passes the light. The light may pass through a pattern before or after the beam splitter to cause the light to become patterned light. Along an optical path of the light after the unidirectional mirror or beam splitter are optics, which may include one or more lens groups. Any of the lens groups may include only a single lens or multiple lenses. One of the lens groups may include at least one moving lens.

The light may pass through an endoscopic probing member, which may include a rigid, light-transmitting medium, which may be a hollow object defining within it a light transmission path or an object made of a light transmitting material, e.g. a glass body or tube. In one embodiment, the endoscopic probing member includes a prism such as a folding prism. At its end, the endoscopic probing member may include a mirror of the kind ensuring a total internal reflection. Thus, the mirror may direct the array of light beams towards a teeth segment or other object. The endoscope probing member thus emits light, which optionally passes through one or more windows and then impinges on to surfaces of intraoral objects.

The light may include an array of light beams arranged in an X-Y plane, in a Cartesian frame, propagating along a Z axis, which corresponds to an imaging axis or viewing axis of the intraoral scanner. As the surface on which the incident light beams hits is an uneven surface, illuminated spots may be displaced from one another along the Z axis, at different (Xᵢ, Yᵢ) locations. Thus, while a spot at one location may be in focus of the confocal focusing optics, spots at other locations may be out-of-focus. Therefore, the light intensity of returned light beams of the focused spots will be at its peak, while the light intensity at other spots will be off peak. Thus, for each illuminated spot, multiple measurements of light intensity are made at different positions along the Z-axis. For each of such (Xᵢ, Yᵢ) location, the derivative of the intensity over distance (Z) may be made, with the Zᵢ yielding maximum derivative, Z₀, being the in-focus distance.

The light reflects off of intraoral objects and passes back through windows (if they are present), reflects off of the mirror, passes through the optical system, and is reflected by the beam splitter onto a detector. The detector is an image sensor having a matrix of sensing elements each representing a pixel of the scan or image. In one embodiment, the detector is a charge coupled device (CCD) sensor. In one embodiment, the detector is a complementary metal-oxide semiconductor (CMOS) type image sensor. Other types of image sensors may also be used for detector. In one embodiment, the detector detects light intensity at each pixel, which may be used to compute height or depth.

Alternatively, in some embodiments an intraoral scanner that uses stereo imaging is used to determine depth information.

As discussed above, in embodiments scanner 20 includes multiple cameras. These cameras may periodically generate intraoral images (e.g., 2D intraoral images), where each of the intraoral images may have a slightly different frame of reference due to the different positions and/or orientations of the cameras generating the intraoral images.

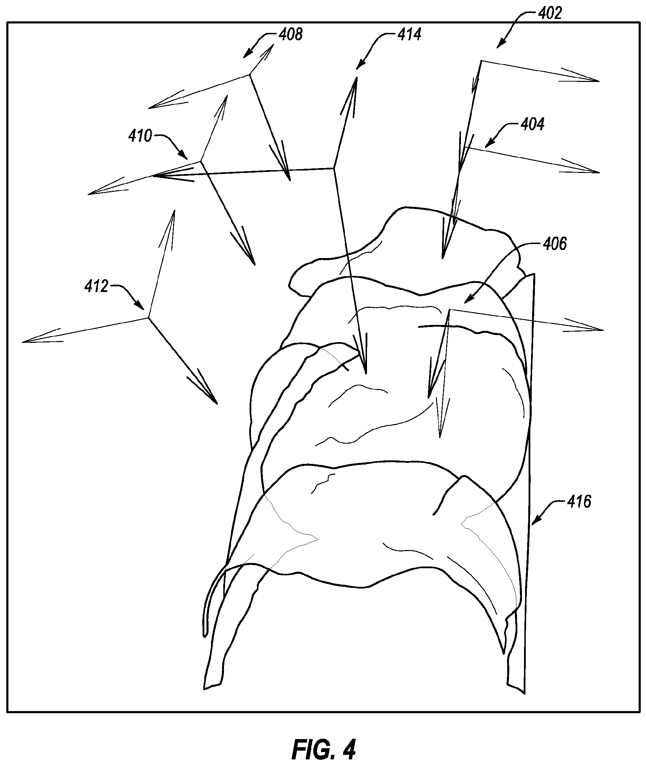

**FIG. 4** illustrates reference frames of multiple cameras of an intraoral scanner relative to a scanned intraoral object 516, in accordance with an embodiment of the present disclosure. In the illustrated example, the scanner includes six cameras, each having a distinct frame of reference 502, 504, 506, 508, 510, 512. In some embodiments, a central or average 514 frame of reference may be computed based on the multiple frames of reference.

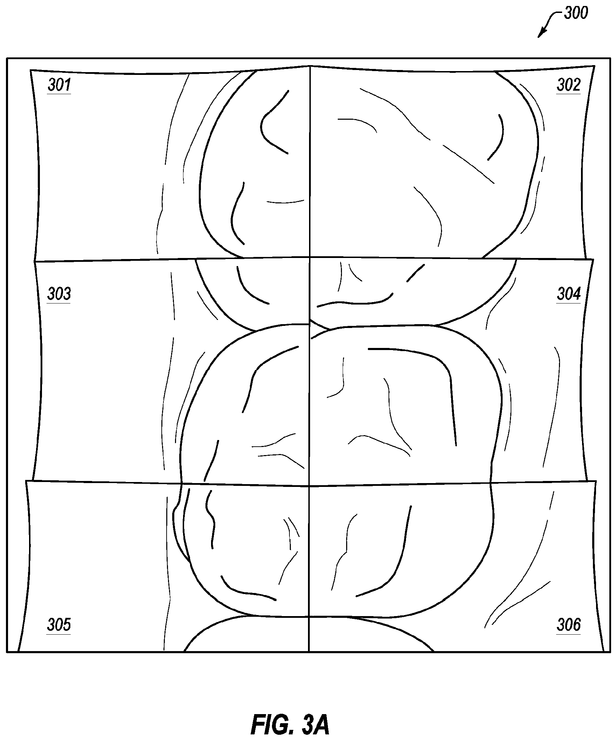

**FIG. 3A** illustrates 2D images (e.g., intraoral images) 301, 302, 303, 304, 305, 306 of a first dental site generated by an array of cameras of an intraoral scanner, in accordance with embodiments of the present disclosure. In one embodiment, the 2D images are generated, respectively, by cameras having the frames of reference shown in **FIG. 4****.**

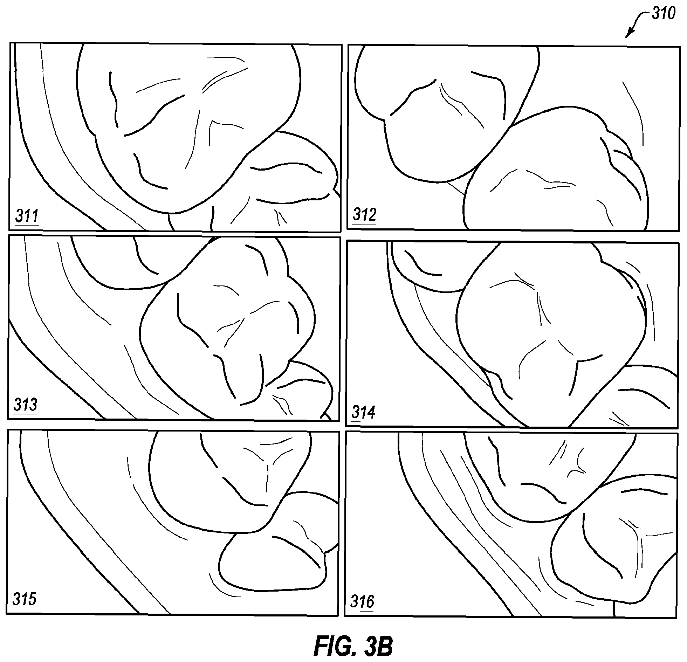

**FIG. 3B** illustrates 2D images 311, 312, 313, 314, 315, 316 of a second dental site generated by an array of cameras of an intraoral scanner, in accordance with embodiments of the present disclosure. In one embodiment, the 2D images are generated, respectively, by cameras having the frames of reference shown in **FIG. 4****.**

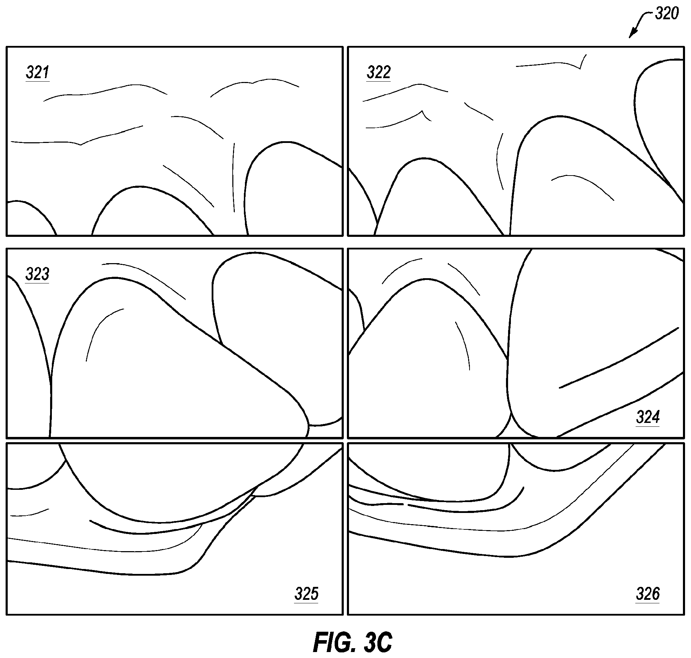

**FIG. 3C** illustrates 2D images 321, 322, 323, 324, 325, 326 of a third dental site generated by an array of cameras of an intraoral scanner, in accordance with embodiments of the present disclosure. In one embodiment, the 2D images are generated, respectively, by cameras having the frames of reference shown in **FIG. 4****.**

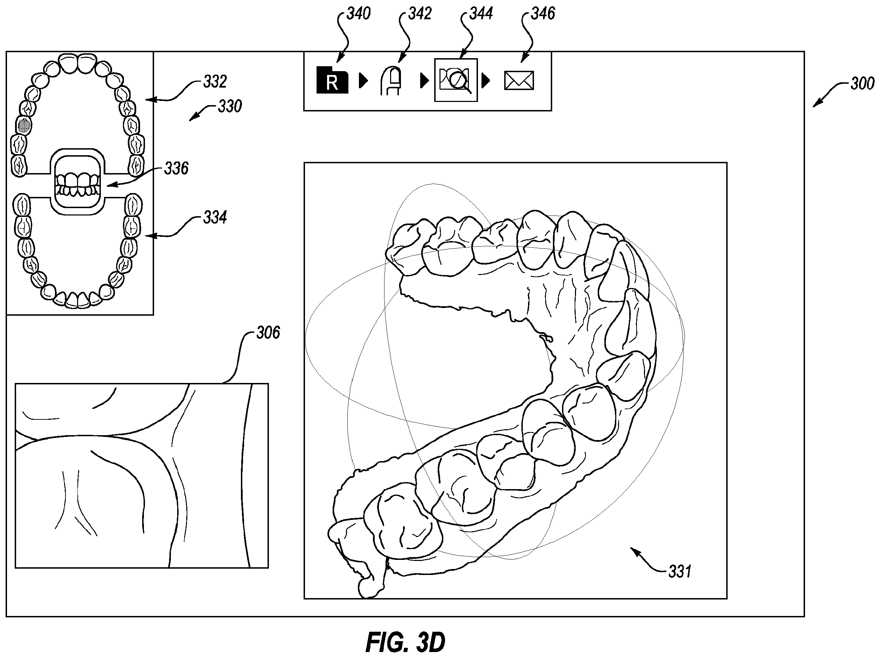

**FIG. 3D** illustrates a view 300 of a graphical user interface of an intraoral scan application that includes a 3D surface 331 and a selected 2D image 306 of a current field of view (FOV) of a camera of an intraoral scanner, in accordance with embodiments of the present disclosure. In the illustrated example, the selected 2D image corresponds to 2D image 306 from the set of 2D images shown in **FIG. 3A****.** The 3D surface 331 is generated by registering and stitching together multiple intraoral scans captured during an intraoral scanning session. As each new intraoral scan is generated, that scan is registered to the 3D surface and then stitched to the 3D surface. Accordingly, the 3D surface becomes more and more accurate with each intraoral scan, until the 3D surface is complete. A 3D model may then be generated based on the intraoral scans.

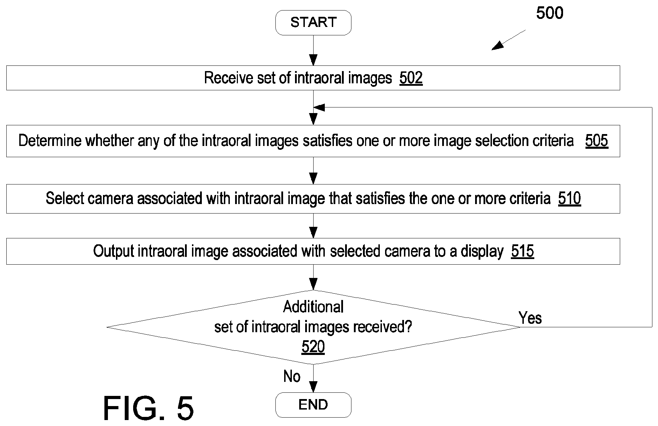

During intraoral scanning, it can be challenging for a user of the intraoral scanner to determine where the FOV of the scanner is currently positioned in the patient's mouth. This is especially true for intraoral scanners that include multiple cameras, where each of the cameras may generate a different 2D image (e.g., a color 2D image) of a different region and/or perspective of a scanned intraoral object. Accordingly, in embodiments a selection or one or more images may be made from multiple 2D images that are generated at or around the same time, each by a different camera. The selected 2D image may then be shown in the GUI. How the 2D image (or images) is/are selected is discussed in greater detail below with reference to **FIGS. 5-10****.**

In some embodiments, a subset of 2D images is selected and then used to generate a single combined 2D image (e.g., a combined viewfinder image). In some embodiments, the combined 2D image is generated without using any 3D surface data of the dental site. For example, the combined 2D image may be generated based on projecting a set of 2D images onto a plane having a predetermined shape, angle and/or distance from a surface of a probe head of an intraoral scanner. Alternatively, 3D surface data may be used to generate a rough estimate of the surface being scanned, and the set of 2D images may be projected onto that rough estimate of the surface being scanned. Alternatively, previous 3D surface data that has already been processed using robust algorithms for accurately determining a shape of the 3D surface may be used along with motion data to estimate surface parameters of a surface onto which the set of 2D images are projected. In any case, the projected 2D images may be merged into the combined image. In embodiments, the combined 2D image is generated using the techniques set forth in U.S. Patent Application No. 17/894,096, filed August 23, 2022.

The GUI for the intraoral scan application may show the selected 2D image 306 in a region of the GUI's display. Sets of 2D images may be generated by the cameras of the intraoral scanner at a frame rate of about 20 frames per second (updated every 50 milliseconds) to about 15 frames per second (updated every 66 milliseconds), and one or more images/cameras is selected from each set. In one embodiment, the 2D images are generated every 20-100 milliseconds.

In one embodiment, as shown, a scan segment indicator 330 may include an upper dental arch segment indicator 332, a lower dental arch segment indicator 334 and a bite segment indicator 336. While the upper dental arch is being scanned, the upper dental arch segment indicator 332 may be active (e.g., highlighted). Similarly, while the lower dental arch is being scanned, the lower dental arch segment indicator 334 may be active, and while a patient bite is being scanned, the bite segment indicator 336 may be active. A user may select a particular segment indicator 332, 334, 336 to cause a 3D surface associated with a selected segment to be displayed. A user may also select a particular segment indicator 332, 334, 336 to indicate that scanning of that particular segment is to be performed. Alternatively, processing logic may automatically determine a segment being scanned, and may automatically select that segment to make it active.

The GUI of the intraoral scan application may further include a task bar with multiple modes of operation or phases of intraoral scanning. Selection of a patient selection mode 340 may enable a doctor to input patient information and/or select a patient already entered into the system. Selection of a scanning mode 342 enables intraoral scanning of the patient's oral cavity. After scanning is complete, selection of a post processing mode 344 may prompt the intraoral scan application to generate one or more 3D models based on intraoral scans and/or 2D images generated during intraoral scanning, and to optionally perform an analysis of the 3D model(s). Examples of analyses that may be performed include analyses to detect areas of interest, to assess a quality of the 3D model(s), and so on.

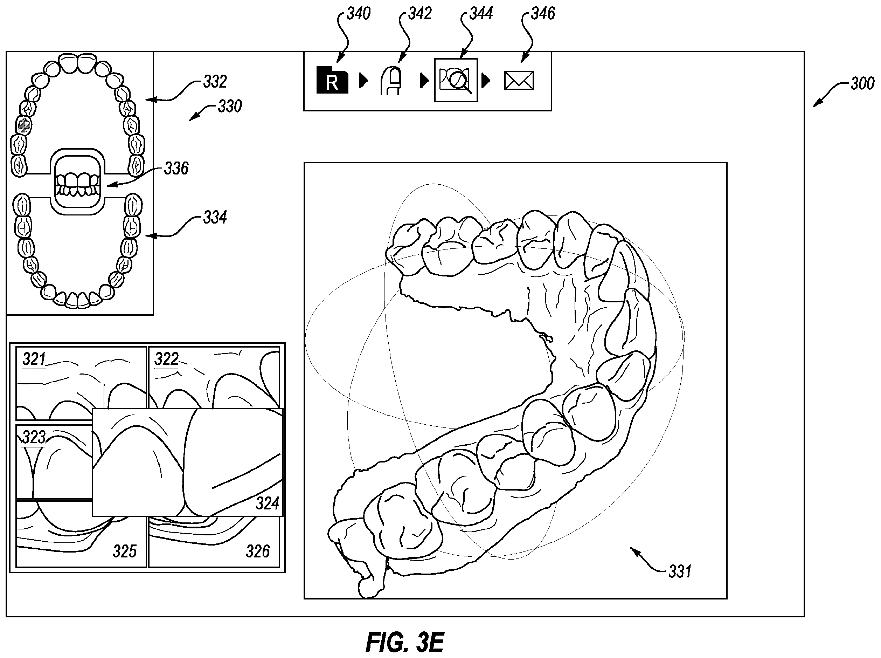

**FIG. 3E** illustrates a view 301 of a graphical user interface of an intraoral scan application that includes a 3D surface and a selected 2D image of a current field of view of an intraoral scanner, in accordance with embodiments of the present disclosure. **FIG. 3E** is substantially similar to **FIG. 3D****,** except in how a selected image from a set of intraoral images is displayed. In **FIG. 3D****,** view 300 shows only a selected image, and does not display non-selected images. In **FIG. 3E****,** on the other hand, view 301 shows each of the images from an image set (in particular from the image set of **FIG. 3C****),** but emphasizes the selected image. In one embodiment, the selected image is emphasized by using a different visualization from a remainder of the images (e.g., the non-selected images). For example, the selected image may be shown with 0% transparency, and other images may be shown with 20-90% transparency. In another example, a zoomed in or larger version of the selected image may be shown, while a zoomed out or smaller version of the non-selected images may be shown, as in **FIG. 3D****.**

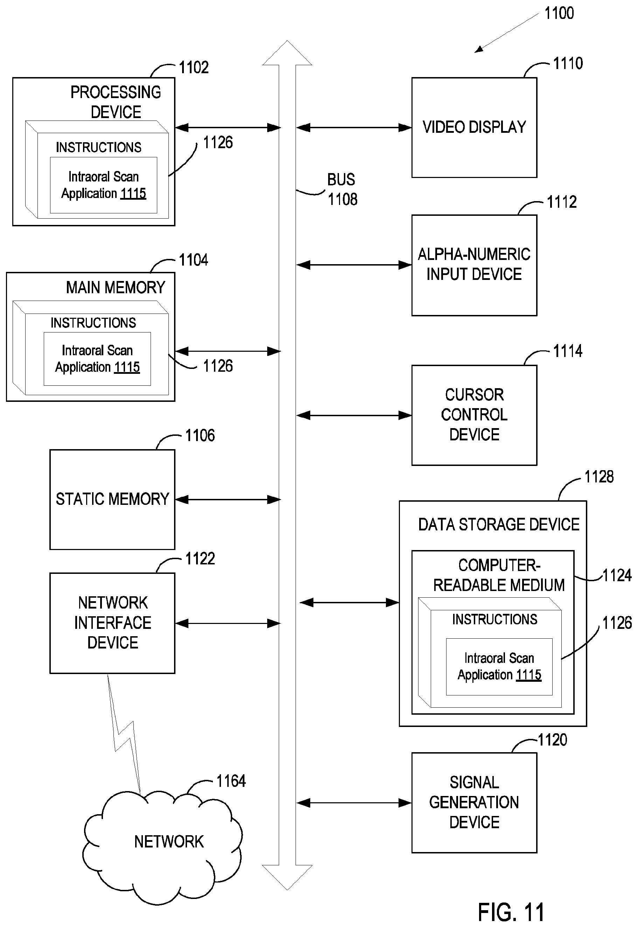

**FIGS. 5-10** are flow charts illustrating various methods related to selection of one or more 2D images from a set of 2D images of an intraoral scanner. Each image in the set of 2D images is generated by a different camera, which may have a unique position and orientation relative to the other cameras. Thus, the various cameras may have different fields of view, which may or may not overlap with the fields of view of other cameras. Each camera may generate images having a different perspective than the other images generated by the other cameras. The methods may be performed by a processing logic that may comprise hardware (e.g., circuitry, dedicated logic, programmable logic, microcode, etc.), software (e.g., instructions run on a processing device to perform hardware simulation), firmware, or a combination thereof. In one embodiment, at least some operations of the methods are performed by a computing device of a scanning system and/or by a server computing device (e.g., by computing device 105 of **FIG. 1** or computing device 1100 of **FIG. 11****).**

**FIG. 5** illustrates a flow chart of an embodiment for a method 500 of selecting an image from a plurality of disparate images generated by cameras of an intraoral scanner, in accordance with embodiments of the present disclosure. The selected image may be, for example, a viewfinder image that shows a current field of view a camera of an intraoral scanner.

At block 502 of method 500, processing logic receives a set of intraoral 2D images. The intraoral 2D images may be color 2D images in embodiments. Alternatively or additionally, the 2D images may be monochrome images, NIR images, or other type of images. Each of the images in the set of images may have been generated by a different camera or cameras at the same time or approximately the same time. For example, the set of images may correspond to images 301-306 of **FIG. 3A** **or** images 311-316 of **FIG. 3B** or images 321-326 of **FIG. 3C****.**

At block 505, processing logic determines whether any of the intraoral images of the set of intraoral images satisfiers one or more image selection criteria. In one embodiment, the image selection criteria comprise a highest score criterion. Scores (also referred to as values) may be computed for each of the images based on one or more properties of the images, and the image having the highest score may satisfy the image selection criteria. Scores may be determined based on a number of pixels or amount of area in an image having a particular classification in some embodiments. Scores for individual images may be adjusted based on scores of one or more surrounding or other images, such as with use of a weighting matrix in some embodiments. In some embodiments, determining whether any of the intraoral images satisfies one or more criteria includes inputting the set of intraoral images into a trained machine learning model that outputs a recommendation for a selection of a camera associated with one of the input images. Other image selection criteria and/or techniques may also be used.

At block 510, processing logic selects the camera associated with the intraoral image that satisfies the one or more criteria. In one embodiment, the image having a highest score is selected. In one embodiment, an image that was recommended for selection by a machine learning model is selected.

At block 515, processing logic outputs the intraoral image associated with the selected camera (e.g., the intraoral image having the highest score) to a display. This may provide a user with information on a current field of view of the selected camera, and in turn of the intraoral scanner (or at least a portion thereof).

At block 520, processing logic may receive an additional set of intraoral images. The initial set of intraoral images may have been generated at a first time during an intraoral scanning session, and a second set of intraoral images may be generated at a later second time during the intraoral scanning session. If a second set of intraoral images is received, the method returns to block 505, and a determination is made as to whether any of the intraoral images of the second set of intraoral images satisfies the one or more image selection criteria. The camera(s) associated with the image(s) that satisfy the one or more criteria may then be selected at block 510. During intraoral scanning, the selected camera may periodically change. This may ensure that the camera that is currently generating the highest quality or most relevant information is selected at any given time in embodiments. IF at block 520 no additional sets of intraoral images are received (e.g., intraoral scanning is complete), the method ends.

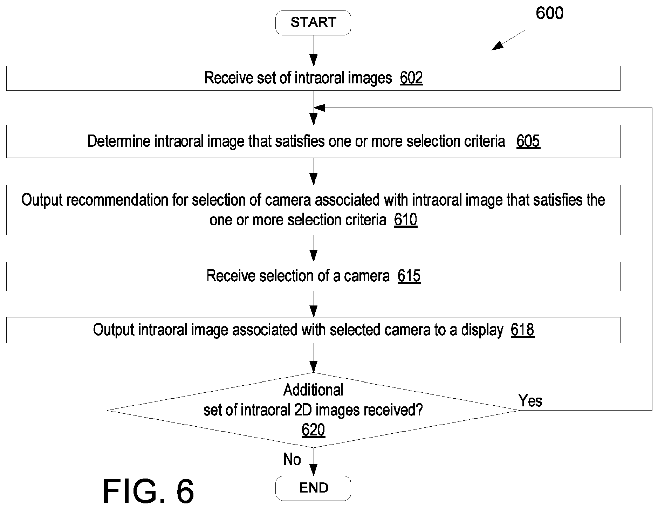

**FIG. 6** illustrates a flow chart of an embodiment for a method 600 of recommending an image from a plurality of disparate images generated by cameras of an intraoral scanner, in accordance with embodiments of the present disclosure. The selected image may be, for example, a viewfinder image that shows a current field of view a cameras of an intraoral scanner.

At block 602 of method 600, processing logic receives a set of intraoral 2D images. The intraoral 2D images may be color 2D images in embodiments. Alternatively or additionally, the 2D images may be monochrome images, NIR images, or other type of images. Each of the images in the set of images may have been generated by a different camera or camera at the same time or approximately the same time. For example, the set of images may correspond to images 301-306 of **FIG. 3A** or images 311-316 of **FIG. 3B** or images 321-326 of **FIG. 3C****.**

At block 605, processing logic determines whether any of the intraoral images of the set of intraoral images satisfies one or more image selection criteria. In one embodiment, the image selection criteria comprise a highest score criterion. Scores may be computed for each of the images based on one or more properties of the images, and the image having the highest score may satisfy the image selection criteria. Scores may be determined based on a number of pixels or amount of area in an image having a particular classification in some embodiments. Scores for individual images may be adjusted based on scores of one or more surrounding or other images, such as with use of a weighting matrix in some embodiments. In some embodiments, determining whether any of the intraoral images satisfies one or more criteria includes inputting the set of intraoral images into a trained machine learning model that outputs a recommendation for a selection of a camera associated with one of the input images. Other image selection criteria and/or techniques may also be used.

At block 610, processing logic outputs a recommendation for selection of a camera associated with an intraoral image that satisfies the one or more selection criteria. The recommendation may be output to a display in embodiments. For example, a prompt may be provided in a GUI of an intraoral scan application. In one embodiment, each of the images from the set of images is displayed in the GUI of the intraoral scan application, and the recommended intraoral image is emphasized (e.g., such as shown in FIG. 3E).

At block 615, processing logic receives selection of one of the intraoral scans, and of the camera associated with that image. The selected image/camera may or may not correspond to the recommended image/camera. A user may select the recommended image or any of the other images. After selection, in some embodiments the non-selected images are no longer shown in the GUI, and only the selected image is shown. The selected image may be enlarged after selection of the image in some embodiments (e.g., to occupy space previously occupied by the non-selected images).

At block 618, processing logic outputs the intraoral image associated with the selected camera (e.g., the intraoral image having the highest score) to the display (e.g., in the GUI). This may provide a user with information on a current field of view of the selected camera, and in turn of the intraoral scanner (or at least a portion thereof).

At block 620, processing logic may receive an additional set of intraoral images. The initial set of intraoral images may have been generated at a first time during an intraoral scanning session, and a second set of intraoral images may be generated at a later second time during the intraoral scanning session. If a second set of intraoral images is received, the method returns to block 605, and a determination is made as to whether any of the intraoral images of the second set of intraoral images satisfies the one or more image selection criteria. The camera(s) associated with the image(s) that satisfy the one or more criteria may then be selected at block 610. During intraoral scanning, the selected camera may periodically change. This may ensure that the camera that is currently generating the highest quality or most relevant information is selected at any given time in embodiments. IF at block 620 no additional sets of intraoral images are received (e.g., intraoral scanning is complete), the method ends.

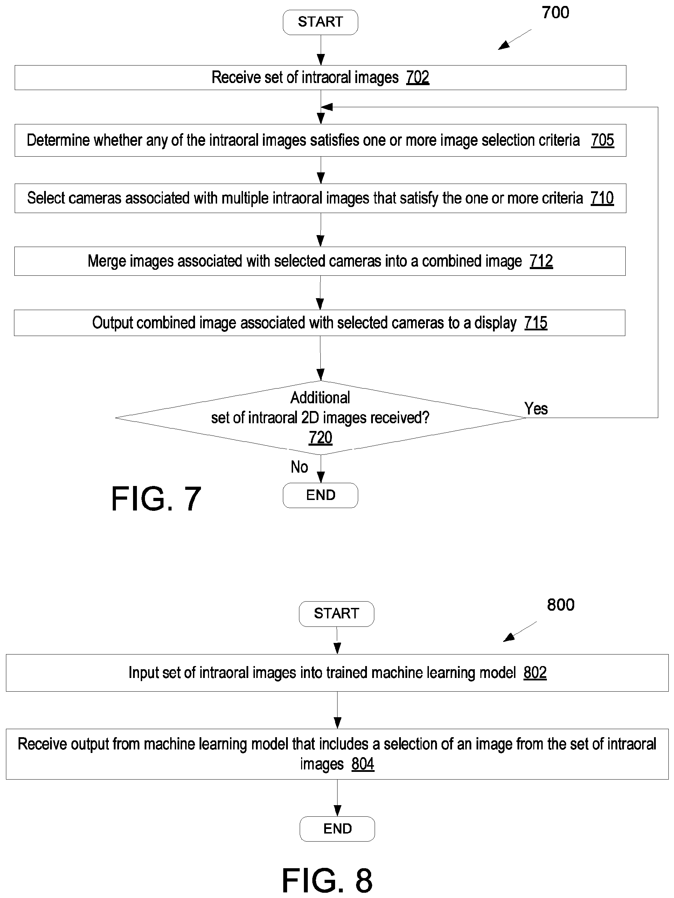

**FIG. 7** illustrates a flow chart of an embodiment for a method 700 of automatically selecting multiple intraoral images to display from a set of intraoral images and generating a combined image from the selected images, in accordance with embodiments of the present disclosure.

At block 702 of method 700, processing logic receives a set of intraoral images. The intraoral images may be color 2D images in embodiments. Alternatively, the intraoral images may be monochrome images, NIR images, or other type of images. Each of the images in the set of images may have been generated by a different camera or camera at the same time or approximately the same time. For example, the set of images may correspond to images 301-306 of **FIG. 3A** **or** images 311-316 of **FIG. 3B** or images 321-326 of **FIG. 3C****.**

At block 705, processing logic determines whether any of the intraoral images of the set of intraoral images satisfies one or more image selection criteria. In one embodiment, the image selection criteria comprise a highest score criterion. Scores may be computed for each of the images based on one or more properties of the images, and the image having the highest score may satisfy the image selection criteria. Scores may be determined based on a number of pixels or amount of area in an image having a particular classification in some embodiments. Scores for individual images may be adjusted based on scores of one or more surrounding or other images, such as with use of a weighting matrix in some embodiments. In some embodiments, determining whether any of the intraoral images satisfies one or more criteria includes inputting the set of intraoral images into a trained machine learning model that outputs a recommendation for a selection of cameras associated with multiple input images. In some embodiments, the selected cameras are adjacent to each other in the intraoral scanner, and the images generated by the selected cameras have at least some overlap.

At block 710, processing logic selects the cameras associated with the intraoral images that satisfy the one or more criteria. In one embodiment, the two or more images having a highest score are selected. In one embodiment, images that were recommended for selection by a machine learning model are selected.

At block 712, processing logic merges together the images associated with the two or more selected cameras into a combined image. In one embodiment, to generate a combined image processing logic determines at least one surface (also referred to as a projection surface) to project the selected intraoral images onto. The different selected images may show a dental site from different angles and positions. Projection of the images from the selected images onto the surface transforms those images into images associated with a reference viewing axis (e.g., of a single virtual camera) that is orthogonal to the surface (or at least a point on the surface) onto which the images are projected. The intraoral images may be projected onto a single surface or onto multiple surfaces. The surface or surfaces may be a plane, a non-flat (e.g., curved) surface, a surface having a shape of a smoothed function, a 3D surface representing a shape of a dental site depicted in the intraoral images, 3D surface that is an estimate of a shape of the dental site, or surface having some other shape. The surface may be, for example, a plane having a particular distance from the intraoral scanner and a particular angle or slope relative to the intraoral scanner's viewing axis. The surface or surfaces may have one or more surface parameters that define the surface, such as distance from the intraoral scanner (e.g., distance from a particular point such as a camera, window or mirror on the intraoral scanner along a viewing axis), angle relative to the intraoral scanner (e.g., angle relative to the viewing axis of the intraoral scanner), shape of the surface, and so on. The surface parameters such as distance from scanner may be pre-set or user selectable in some embodiments. For example, the distance may be a pre-set distance of 1-15 mm from the intraoral scanner. In one embodiment, the surface onto which the images are projected is a plane that is orthogonal to a viewing axis of the intraoral scanner. In one embodiment, processing logic projects a 3D surface or an estimate of a 3D surface based on recently received intraoral scans onto the plane to generate a height map. Height values may be used to help select image data to use for pixels of a combined image.

In some embodiments, different regions of an image are projected onto different surfaces. For example, if it is known that a first region of a dental site is approximately at a first distance from the intraoral scanner and a second region of the dental site is approximately at a second distance from the intraoral scanner, then a first region of an image that depicts the first region of the dental site may be projected onto a first surface having the first distance from the intraoral scanner and a second region of the image that depicts the second region of the dental site may be projected onto a second surface having the second distance from the intraoral scanner. In some embodiments, different images are projected onto different surfaces. In some embodiments, one or more of the images are projected onto multiple surfaces, and a different combined image is generated for each of the surfaces. A best combined image (associated with a particular surface) may then be selected based on an alignment of edges and/or projected image borders between the projections of the intraoral images onto the respective surfaces. The surface that resulted in a closest alignment of edges and/or borders between the intraoral images may be selected as the surface to use for generation of the combined image, for example.

In one embodiment, processing logic determines, for each selected intraoral image of the set of intraoral images, projection parameters for projecting the intraoral image onto the at least one surface. Each camera may have a unique known orientation relative to the surface, resulting in a unique set of projection parameters for projecting images generated by that camera onto a determined surface.

In one embodiment, processing logic projects the selected intraoral images onto the at least one surface. Each projection of an intraoral image onto the surface may be performed using a unique set of projection parameters.

In one embodiment, processing logic generates a combined intraoral image based on merging the projected intraoral images. Merging the images into a single combined image may include performing image registration between the images and stitching the images together based on a result of the registration. In one embodiment, the intraoral images were projected onto a height map. Processing logic may determine, for every point on the height map, and for every image that provides data for that point, an angle between a chief ray of a camera that generated the image and an axis orthogonal to the height map. Processing logic may then select a value for that point from the image associated with the camera having a smallest angle between the chief ray and the axis orthogonal to the height map. In other words, processing logic takes, for every point on the height map, its value from the camera for which its camera direction (chief ray) is the closest to the direction from the camera pinhole to the point on the height map.

Merging the selected images may include, for example, simply aligning the image boundaries of the images with one another (e.g., by tiling the images in a grid). Merging the set of images may additionally or alternatively include performing one or more blending operations between the images. For example, in some instances the lines and/or edges within a first image may not line up with lines and/or edges in an adjacent second image being merged with the first image. A weighted or unweighted average may be used to merge the edges and/or lines within the images. In one embodiment, an unweighted average is applied to the center of an overlap between two adjacent images. Processing logic can smoothly adjust the weightings to apply in generating the average of the two overlapping intraoral images based on a distance from the center of the overlapped region. As points that are closer to an outer boundary of one of the images are considered, that one image may be assigned a lower weight than the other image for averaging those points. In one embodiment, Poisson blending is performed to blend the projected intraoral images together.

In one embodiment, processing logic determines outer boundaries of each selected intraoral image that has been projected onto the surface. Processing logic then determines one or more image boundaries in a first image of the selected intraoral images that fail to line up in an overlapping region with one or more image boundaries in an adjacent second image of the selected intraoral images. Processing logic then adjusts at least one of the first image or the second image to cause the one or more image boundaries in the first intraoral image to line up with the one or more image boundaries in the adjacent second intraoral image. This may include, for example, re-scaling one or both of the images, stretching or compressing one or both of the images along one or more axis, and so on.

In one embodiment, merging of the projected images includes deforming one or more of the images to match gradients at the boundaries of adjacent images. For example, some regions of the initially projected images may not register properly due to the various camera angles or perspectives associated with the images. In one implementation, processing logic uses a global optimization method to identify the appropriate image deformation required to match the boundaries of adjacent images. Once the deformation has been identified, processing logic can apply a deformation to one or more of the projected images to deform those images. Processing logic may then blend the images (one or more of which may be a deformed image) to produce a final combined image. In one implementation, processing logic uses Poisson blending to use target gradients from non-blended images to produce a blended image with gradients that best match those target gradients.

Some regions of the projected images may not register properly due to the various camera angles or perspectives associated with those images. Accordingly, it may be necessary to register and/or deform the projected images to match gradients at the boundaries of adjacent images. The deformation may include several distinct steps, such as a global optimization followed by a local optimization along the image boundaries only. In one example, a global optimization technique (such as projective image alignment by using Enhanced Correlation Coefficient, or ECC, maximization) can be used to identify the appropriate image deformation required to match the boundaries of adjacent images. After applying the deformation identified in the global optimization, the image boundaries may still not match. Next a local optimization along the image boundaries only can be used to identify an appropriate deformation along the image boundaries required to match the boundaries of adjacent images. The identified boundary deformation can be analytically extended to the interior of each image to deform the images in a smooth and realistic manner. The resulting deformed images can be blended to produce a combined image.

At block 715, processing logic outputs the combined intraoral image associated with the selected cameras to a display. The combined intraoral image may be, for example, a viewfinder image that shows a field of view of the intraoral scanner.

At block 720, processing logic determines whether an additional set of intraoral images has been received. If so, the method returns to block 705 and operations 705-715 are repeated for the new set of intraoral images. This process may continue until at block 720 a determination is made that no new intraoral images have been received, at which the method may end. The intraoral scanner may periodically or continuously generate new sets of intraoral images, which may be used to select cameras and generate combined 2D images in real time or near-real time. Thus, the user of the intraoral scanner may be continuously updated with a combined image showing the current field of view of a subset of cameras of the intraoral scanner.

**FIG. 8** illustrates a flow chart of an embodiment for a method 800 of determining which image from a set of images to select for display using a trained machine learning model, in accordance with embodiments of the present disclosure. Method 800 may be performed, for example, at block 505 of method 500, at block 605 of method 600, at block 705 of method 700, and so on. At block 802 of method 800, a received set of intraoral images is input into a trained machine learning model. The trained machine learning model may be, for example, a neural network such as a deep neural network, convolutional neural network, recurrent neural network, etc. Other types of machine learning models such as a support vector machine, random forest model, regression model, and so on may also be used. The machine learning model may have been trained using labeled sets of intraoral images, where for each set of intraoral images the labels indicate one or more images/cameras that should be selected.

At block 804, processing logic receives an output from the trained machine learning model, where the output includes a selection/recommendation for selection of an image (or multiple images) from the set of intraoral images that were input into the trained machine learning model.

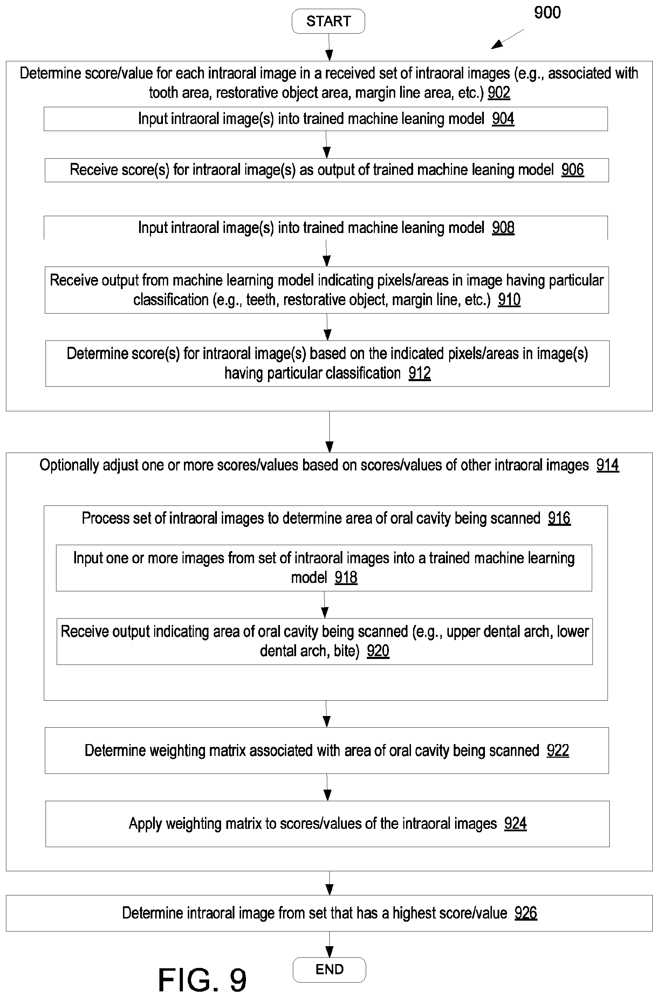

**FIG. 9** illustrates a flow chart of an embodiment for a method 900 of determining which image from a set of images meets one or more image selection criteria, and ultimately of determining which image to select/recommend for display and/or of determining which camera to select/recommend, in accordance with embodiments of the present disclosure. Method 900 may be performed, for example, at block 505 of method 500, at block 605 of method 600, at block 705 of method 700, and so on.

At block 902 of method 900, processing logic determines a score (or value) for each intraoral image in a received set of intraoral images. The score may be determined based on, for example, properties such as image blurriness, area of image depicting a tooth area, area of image depicting a restorative object, area of image depicting a margin line, image contrast, lighting conditions, and so on.

In one embodiment, each intraoral image from the set of intraoral images is input into a trained machine learning model. The machine learning model may be a neural network (e.g., deep neural network, convolutional neural network, recurrent neural network, etc.), support vector machine, random forest model, or other type of model. In one embodiment, intraoral images are downsampled before being input into the model. The trained machine learning model may have been trained to grade images (e.g., to assign scores to images). For example, an application engineer may have manually labeled images from many sets of intraoral images, where for each set an optimal image was indicated. The learning would minimize the distance between an output vector of the machine learning model and a vector containing a 1 for an indicated optimal camera and 0s for other cameras. For example, for a given set of 6 images (e.g., for a scanner having 6 cameras), a label for the set of images may be [0,1,0,0,0,0], which indicates that the second camera is the optimal camera for the set.

In one embodiment, each intraoral image is separately input into the machine learning model, which outputs a score for that input image. In one embodiment, images are downsampled before being input into the machine learning model. In one embodiment, two or more intraoral images are input together into the machine learning model. For multiple images input into the machine learning model, the machine learning model may output a score for just one of the images, or a separate score for each of the images. For example, a primary image to be scored may be input into the machine learning model together with one or more secondary images. Scores may not be generated for the secondary images, but the data from the secondary images may be used by the machine learning model in determining the score for the primary image. In one embodiment, the primary image is a color image, and the secondary images include color and/or NIR images. In another example, the entire set of intraoral images may be input into the machine learning model together, and a separate score may be output for each of the input images. The score for each image may be influenced by data from the given image as well as by data from other images of the set of images.

At block 906, processing logic outputs scores for one or more of the intraoral images from the set. In one embodiment, a score assigned to an image has a value of 0 to 1, where higher scores represent a higher importance of a camera that generated the image. In one embodiment, the full set of images is input into the trained machine learning model, and the model outputs a feature vector comprising a value of 0-1 for each camera.

In one embodiment, at block 906 processing logic inputs each of the intraoral images into a trained machine learning model (e.g., one at a time). At block 910, for each image input into the machine learning model, the machine learning model performs pixel-level or patch-level (e.g., where a patch includes a group of pixels) classification of the contents of the image. This may include performing segmentation of the image in some embodiments. In embodiments, the trained machine learning model classifies pixels/patches into different dental object classes, such as teeth, gums, tongue, restorative object, preparation tooth, margin line, and so on. In one embodiment, the trained machine learning model classifies pixels/patches into teeth and not teeth.

At block 910, processing logic may receive outputs from the machine learning model, where each output indicates the classifications of pixels and/or areas in an image. In one embodiment, the output for an image is a mask or map, where the mask or map may have a same resolution (e.g., same number of pixels) as the image. Each pixel of the mask or map may have a first value if it has been assigned a first classification, a second value if it has been assigned a second classification, and so on. For example, the machine learning model may output a binary mask that includes a 1 for each pixel classified as teeth and a 0 for each pixel not classified as teeth. In one embodiment, each pixel in the output may have an assigned value between -1 and 1, where -1 indicates a 0% probability of belonging to a tooth, a 0 represents a 50% probability of belonging to a tooth, and a 1 represents a 100% probability of belonging to a tooth.

At block 912, processing logic may determine scores for each image based on the output of the trained machine learning model for that image. In one embodiment, processing logic determines a size of an area (e.g., a number of pixels) in the image that have been assigned a particular classification (e.g., classified as teeth, or classified as a restorative object, or classified as a preparation tooth, or classified as a margin line), and computes the score based on the size of the area assigned the particular classification. There may be a direct linear or non-linear correlation between a size of the area having the classification and the score for an image in some embodiments. In one embodiment, the score is based on a ratio of the number of pixels having a particular classification (e.g., teeth) to a total number of pixels in the image.

In some embodiments, a camera associated with an image having a highest raw score may not be an optimal camera. Accordingly, in some embodiments scores for images are adjusted based on the scores of one or more other (e.g., adjacent or surrounding) images. In some instances the existence or absence of tooth data in one or more images may be used to infer information about position of a probe head of an intraoral scanner in a patient's mouth. For example, in the 6-camera image set shown in FIG. 3B, it can be seen that all cameras located vertically show relatively large teeth area. Accordingly, processing logic can conclude that the probe is inserted inside of a patient's mouth and the font cameras are located over the patient's distal molars. Accordingly, assuming that distal molar scanning is important, processing logic can select one of the front cameras, even if the individual scores of those cameras may be lower than the individual scores of other cameras.

In another example, the 6-camera image set shown in FIG. 3C shows that the front camera (corresponding to the bottom images) barely captured any teeth. Accordingly, processing logic can conclude that the probe is not located deep inside of the patient's mouth. Accordingly, the middle camera(s) may be selected.

At block 914, processing logic optionally adjusts the scores of one or more images based on the scores of other (e.g., adjacent or surrounding) images and/or based on other information discerned about the position of the scanner probe in a patient's mouth. In some embodiments, the scores of one or more images are adjusted based on a weight matrix (also referred to as a weighting matrix). In some embodiments, the weight matrix is static, and the same weight matrix is used for different situations. In other embodiments, a weight matrix may be selected based on one or more criteria, such as based on a determined position of the probe in the patient's mouth, based on a determined scanning role or segment currently being scanned, and so on.

In one embodiment, the scores for the set of images are represented as a vector C (e.g., a 6-vector if six cameras are used). The vector C may then be multiplied by a weight matrix W, which may be a square matrix with a number of rows and columns equal to the length of the vector C. A bias vector b, which may have a same length as the vector C, may then be subtracted from the result of the matrix multiplication. The bias vector b may be fixed, or may be selected based on one or more criteria (e.g., the same or different criteria from those optionally used to select the weight matrix). The scores may be updated according to the following equation in embodiments: $R = WC - b$ Where *R* is the adjusted vector that includes the adjusted scores for each of the images in the set of intraoral images.

In embodiments, the elements of the weight matrix may be determined by preparing a data set of examples, where each one includes camera image sets along with the camera identifier of the desired camera to be displayed for that set as decided by a clinical user or application engineer. Learning can be performed per camera, in which the camera selected will get a value of 1 (in R) and the non-selected images will get a value of 0 (in R). Multiple different learning algorithms may be applied, such as a Perceptron learning algorithm. In some embodiments, the camera organization for the intraoral scanner is left/right symmetrical. Accordingly, in some embodiments, the weight matrices are configured such that weights are left/right symmetrical to reflect the symmetrical arrangement of the cameras.

In some embodiments, the weight matrix is configurational. In some embodiments, the weight matrix is selectable based on a different scanning purpose. For example, different dental objects may be more or less important for scanning performed for restorative procedures with respect to scanning performed for orthodontic procedures. Accordingly, in embodiments a doctor or user may input information on a purpose of scanning (e.g., select restorative or orthodontic), and a weight matrix may be selected based on the user input. In some embodiments, different weight matrices are provided for scanning of an upper dental arch, a lower dental arch, and a patient bite.

In one embodiment, at block 916 processing logic processes the set of intraoral images to determine an area of the oral cavity that is being scanned. For example, processing logic may process the set of images to determine whether an upper dental arch, a lower dental arch, or a patient bite is being scanned.

A scanning process usually has several stages - so-called roles (also referred to as scanning roles). Three major roles are upper jaw role (also referred to as upper dental arch role), lower jaw role (also referred to as lower dental arch role) and bite role. The bite role refers to a role for a relative position of the upper jaw and lower jaw while the jaw is closed. In some embodiments, a user of the scanner chooses a target role by means of the user interface of the intraoral scan application. In some embodiments, processing logic automatically identifies the role while scanning. In some embodiments, processing logic automatically determines whether a user is currently scanning teeth on an upper jaw (upper jaw role), teeth on a lower jaw (lower jaw role), or scanning both teeth on the upper and lower jaw while the patient's jaw is closed (bite role).

In some embodiments, a separate role is assigned to each preparation tooth and/or other restorative object on a dental arch. Thus, roles may include an upper jaw role, a lower jaw role, a bite role, and one or more preparation roles, where a preparation role may be associated with a preparation tooth or another type of preparation or restorative object. In addition to automatically identifying the upper jaw role, lower jaw role, and bite role, processing logic may also automatically identify preparation roles from intraoral scan data (e.g., 2D intraoral images), 3D surfaces and/or 3D models. A preparation may be associated with both a jaw role (e.g., an upper jaw role or a lower jaw role) and a preparation role in some embodiments.

In some embodiments, processing logic uses machine learning to detect whether intraoral scans depict an upper dental arch (upper jaw role), a lower dental arch (lower jaw role), or a bite (bite role). In some embodiments, processing logic uses machine learning to detect whether intraoral scans depict an upper dental arch (upper jaw role), a lower dental arch (lower jaw role), a bite (bite role), and/or a preparation (preparation role). As intraoral scan data is generated, intraoral scans from the intraoral scan data and/or 2D images from the intraoral scan data may be input into a trained machine learning model at block 918 that has been trained to identify roles. At block 920, the trained machine learning model may then output a classification of a role (or roles) for the intraoral scan data, indicating an area of the oral cavity being scanned and/or a current scanning role (e.g., upper dental arch, lower dental arch, patient bit, etc.). In some embodiments, roles and/or restorative objects are identified as set forth in U.S. Application No. 17/230,825, filed April 14, 2021.

In one embodiment, at block 922 processing logic determines a weighting matrix associated with an area of the oral cavity being scanned (e.g., with a current scanning role). At block 924, processing logic may apply the weighting matrix to modify the scores of the images in the set of intraoral images, as set forth above.

At block 926, processing logic may determine an intraoral image from the set of intraoral images that has the highest score or value (optionally after performing weighting/adjustment of the scores).

As discussed hereinabove, trained machine learning models may be used in embodiments to perform one or more tasks, such as object identification, pixel-level classification of images, scanning role identification, image selection, and so on. For example, machine learning models may be trained to perform one or more classifying, segmenting, detection, recognition, image generation, prediction, parameter generation, etc. tasks for intraoral scan data (e.g., 3D scans, height maps, 2D color images, NIRI images, etc.). Multiple different machine learning model outputs are described herein. Particular numbers and arrangements of machine learning models are described and shown. However, it should be understood that the number and type of machine learning models that are used and the arrangement of such machine learning models can be modified to achieve the same or similar end results. Accordingly, the arrangements of machine learning models that are described and shown are merely examples and should not be construed as limiting.

In embodiments, one or more machine learning models are trained to perform one or more of the below tasks. Each task may be performed by a separate machine learning model. Alternatively, a single machine learning model may perform each of the tasks or a subset of the tasks. Additionally, or alternatively, different machine learning models may be trained to perform different combinations of the tasks. In an example, one or a few machine learning models may be trained, where the trained ML model is a single shared neural network that has multiple shared layers and multiple higher level distinct output layers, where each of the output layers outputs a different prediction, classification, identification, etc. The tasks that the one or more trained machine learning models may be trained to perform are as follows:
I) Scan view classification - this can include classifying intraoral scans or sets of intraoral scans as depicting a lingual side of a jaw, a buccal side of a jaw, or an occlusal view of a jaw. Other views may also be determinable, such as right side of jaw, left side of jaw, and so on. Additionally, this can include identifying a molar region vs. a bicuspid region, identifying mesial surfaces, distal surfaces and/or occlusal surfaces, and so on. This information may be used to determine an area of the oral cavity being scanned, and optionally to select a weight matrix.
II) Image quality ranking - this can include assigning one or more scanning quality metric values to individual intraoral images from a set of intraoral images. This information can be used to select a camera to use for viewfinder images.
III) Intraoral area of interest (AOI) identification - this can include performing pixel-level or patch-level identification/classification of intraoral areas of interest on one or more images of a set of intraoral images. Examples of AOls include voids, conflicting surfaces, blurry surfaces, surfaces with insufficient data density, surfaces associated with scanning quality metric values that are below a threshold, and so on. This information can be used to select a camera to use for viewfinder images.
IV) Generation of intraoral 2D images - this can include receiving an input of multiple 2D images taken by different cameras at a same time or around a same time and generating a combined intraoral 2D image that includes data from each of the intraoral 2D images. The cameras may have different orientations, making merging of the intraoral 2D images non-trivial.
V) Scanning role identification - this can include determining whether an upper dental arch, lower dental arch, patient bite or preparation tooth is presently being scanned.
VI) Restorative object detection - this can include performing pixel level identification/classification and/or group/patch-level identification/classification of each image in a set of intraoral images to identify/classify restorative objects in the images.
VII) Margin line detection - this can include performing pixel level identification/classification and/or group/patch-level identification/classification of each image in a set of intraoral images to identify/classify margin lines in the images.

One type of machine learning model that may be used to perform some or all of the above asks is an artificial neural network, such as a deep neural network. Artificial neural networks generally include a feature representation component with a classifier or regression layers that map features to a desired output space. A convolutional neural network (CNN), for example, hosts multiple layers of convolutional filters. Pooling is performed, and non-linearities may be addressed, at lower layers, on top of which a multi-layer perceptron is commonly appended, mapping top layer features extracted by the convolutional layers to decisions (e.g. classification outputs). Deep learning is a class of machine learning algorithms that use a cascade of multiple layers of nonlinear processing units for feature extraction and transformation. Each successive layer uses the output from the previous layer as input. Deep neural networks may learn in a supervised (e.g., classification) and/or unsupervised (e.g., pattern analysis) manner. Deep neural networks include a hierarchy of layers, where the different layers learn different levels of representations that correspond to different levels of abstraction. In deep learning, each level learns to transform its input data into a slightly more abstract and composite representation. In an image recognition application, for example, the raw input may be a matrix of pixels; the first representational layer may abstract the pixels and encode edges; the second layer may compose and encode arrangements of edges; the third layer may encode higher level shapes (e.g., teeth, lips, gums, etc.); and the fourth layer may recognize a scanning role. Notably, a deep learning process can learn which features to optimally place in which level on its own. The "deep" in "deep learning" refers to the number of layers through which the data is transformed. More precisely, deep learning systems have a substantial credit assignment path (CAP) depth. The CAP is the chain of transformations from input to output. CAPs describe potentially causal connections between input and output. For a feedforward neural network, the depth of the CAPs may be that of the network and may be the number of hidden layers plus one. For recurrent neural networks, in which a signal may propagate through a layer more than once, the CAP depth is potentially unlimited.

Training of a neural network may be achieved in a supervised learning manner, which involves feeding a training dataset consisting of labeled inputs through the network, observing its outputs, defining an error (by measuring the difference between the outputs and the label values), and using techniques such as deep gradient descent and backpropagation to tune the weights of the network across all its layers and nodes such that the error is minimized. In many applications, repeating this process across the many labeled inputs in the training dataset yields a network that can produce correct output when presented with inputs that are different than the ones present in the training dataset. In high-dimensional settings, such as large images, this generalization is achieved when a sufficiently large and diverse training dataset is made available.

For each machine learning model to be trained, a training dataset containing hundreds, thousands, tens of thousands, hundreds of thousands or more images should be used to form a training dataset. In one embodiment, generating one or more training datasets includes gathering one or more sets of intraoral images with labels. The labels that are used may depend on what a particular machine learning model will be trained to do. For example, to train a machine learning model to perform classification of teeth, a training dataset may images with pixel-level labels of teeth and/or other dental objects.

Processing logic may gather a training dataset comprising intraoral images having one or more associated labels. One or more images may be resized in embodiments. For example, a machine learning model may be usable for images having certain pixel size ranges, and one or more image may be resized if they fall outside of those pixel size ranges. The images may be resized, for example, using methods such as nearest-neighbor interpolation or box sampling. The training dataset may additionally or alternatively be augmented. Training of large-scale neural networks generally uses tens of thousands of images, which are not easy to acquire in many real-world applications. Data augmentation can be used to artificially increase the effective sample size. Common techniques include random rotation, shifts, shear, flips and so on to existing images to increase the sample size.

To effectuate training, processing logic inputs the training dataset(s) into one or more untrained machine learning models. Prior to inputting a first input into a machine learning model, the machine learning model may be initialized. Processing logic trains the untrained machine learning model(s) based on the training dataset(s) to generate one or more trained machine learning models that perform various operations as set forth above.

Training may be performed by inputting one or more of the images into the machine learning model one at a time or in sets. Each input may include data from an image (or set of images), and optionally 3D intraoral scans from the training dataset.

The machine learning model processes the input to generate an output. An artificial neural network includes an input layer that consists of values in a data point (e.g., intensity values and/or height values of pixels in a height map). The next layer is called a hidden layer, and nodes at the hidden layer each receive one or more of the input values. Each node contains parameters (e.g., weights) to apply to the input values. Each node therefore essentially inputs the input values into a multivariate function (e.g., a non-linear mathematical transformation) to produce an output value. A next layer may be another hidden layer or an output layer. In either case, the nodes at the next layer receive the output values from the nodes at the previous layer, and each node applies weights to those values and then generates its own output value. This may be performed at each layer. A final layer is the output layer.

Processing logic may then compare the generated output to the known label that was included in the training data item. Processing logic determines an error (i.e., a classification error) based on the differences between the output probability map and/or label(s) and the provided probability map and/or label(s). Processing logic adjusts weights of one or more nodes in the machine learning model based on the error. An error term or delta may be determined for each node in the artificial neural network. Based on this error, the artificial neural network adjusts one or more of its parameters for one or more of its nodes (the weights for one or more inputs of a node). Parameters may be updated in a back propagation manner, such that nodes at a highest layer are updated first, followed by nodes at a next layer, and so on. An artificial neural network contains multiple layers of "neurons", where each layer receives as input values from neurons at a previous layer. The parameters for each neuron include weights associated with the values that are received from each of the neurons at a previous layer. Accordingly, adjusting the parameters may include adjusting the weights assigned to each of the inputs for one or more neurons at one or more layers in the artificial neural network.

Once the model parameters have been optimized, model validation may be performed to determine whether the model has improved and to determine a current accuracy of the deep learning model. After one or more rounds of training, processing logic may determine whether a stopping criterion has been met. A stopping criterion may be a target level of accuracy, a target number of processed images from the training dataset, a target amount of change to parameters over one or more previous data points, a combination thereof and/or other criteria. In one embodiment, the stopping criteria is met when at least a minimum number of data points have been processed and at least a threshold accuracy is achieved. The threshold accuracy may be, for example, 70%, 80% or 90% accuracy. In one embodiment, the stopping criteria is met if accuracy of the machine learning model has stopped improving. If the stopping criterion has not been met, further training is performed. If the stopping criterion has been met, training may be complete. Once the machine learning model is trained, a reserved portion of the training dataset may be used to test the model.

In some embodiments, while using the above discussed camera selection techniques, processing logic may experience camera transition jitter (e.g., where a selected camera switches too frequently). For example, it may happen that two resulting camera/image scores have close values. This may cause the camera selection to jump back and forth between the two cameras during scanning. To alleviate such rapid switching between camera selection, processing logic may apply a threshold to introduce hysteresis that can reduce jerkiness or frequent camera selection switching. For example, a threshold may be set such that a new camera is selected when the difference between the score for the image of the new camera and the score for the image of the previously selected camera exceeds a difference threshold. Alternatively, use of a recurrent neural network (RNN) that takes into account prior data may alleviate frequent camera selection switching and/or jitter. To train such an RNN, the RNN may be trained on sequences of images, and some penalty may be introduced for each jump between frames (e.g., between sets of images).

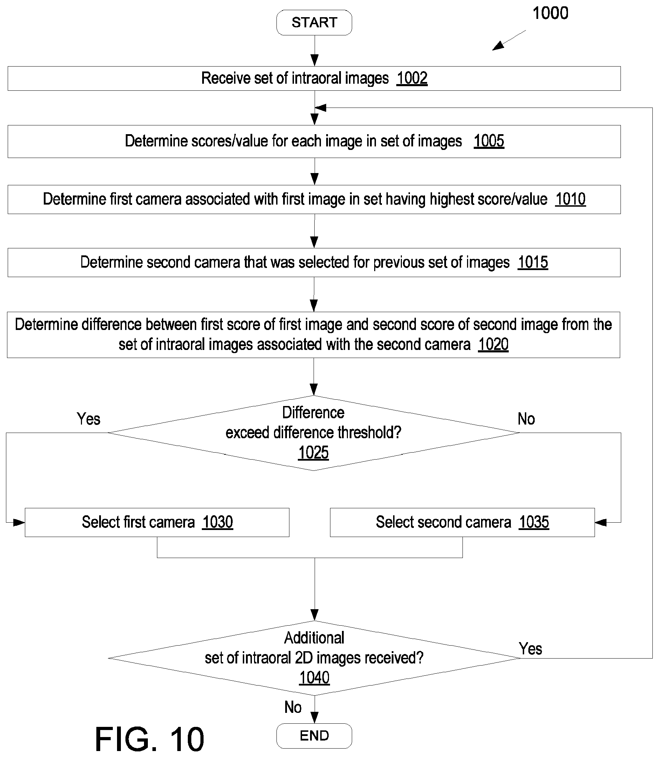

**FIG. 10** illustrates a flow chart of an embodiment for a method 1000 of automatically selecting an intraoral image to display from a set of intraoral images, taking into account selections from prior sets of intraoral images, in accordance with embodiments of the present disclosure. At block 1002 of method 1000, processing logic receives a set of intraoral 2D images. The intraoral 2D images may be color 2D images in embodiments. Alternatively, or additionally, the 2D images may be monochrome images, NIR images, or other type of images. Each of the images in the set of images may have been generated by a different camera or camera at the same time or approximately the same time. For example, the set of images may correspond to images 301-306 of **FIG. 3A** or images 311-316 of **FIG. 3B** or images 321-326 of **FIG. 3C****.**

At block 1005, processing logic determines whether any of the intraoral images of the set of intraoral images satisfies one or more image selection criteria. In one embodiment, the image selection criteria comprise a highest score criterion. Scores may be computed for each of the images based on one or more properties of the images, and the image having the highest score may satisfy the image selection criteria. Scores may be determined based on a number of pixels or amount of area in an image having a particular classification in some embodiments. Scores for individual images may be adjusted based on scores of one or more surrounding or other images, such as with use of a weighting matrix in some embodiments. In some embodiments, determining whether any of the intraoral images satisfies one or more criteria includes inputting the set of intraoral images into a trained machine learning model that outputs a recommendation for a selection of a camera associated with one of the input images. Other image selection criteria and/or techniques may also be used.

At block 1010, processing logic determines a first camera associated with a first image in the set of intraoral images that has a highest score (optionally after adjusting the scoring such as with a weight matrix). At block 1015, processing logic determines a second camera that was selected for a previous set of images. Processing logic determines a score associated with a second image from the current set of images that is associated with the second camera. At block 1020, processing logic determines a difference between a first score of the first image and a second score of the second image.

At block 1025, processing logic determines whether or not the determined difference exceeds a difference threshold. If the difference does exceed the difference threshold, the method proceeds to block 1030 and the first camera is selected for the current set of images. If the difference does not exceed the difference threshold, the method continues to block 1035 and processing logic selects the second camera (that was selected for the previous set of images). The image associated with the selected camera may then be output to a display.

At block 1040, processing logic may receive an additional set of intraoral images. The initial set of intraoral images may have been generated at a first time during an intraoral scanning session, and a second set of intraoral images may be generated at a later second time during the intraoral scanning session. If a second set of intraoral images is received, the method returns to block 1005, and the operations of blocks 1005-1035 are repeated. If at block 1040 no additional sets of intraoral images are received (e.g., intraoral scanning is complete), the method ends.

**FIG. 11** illustrates a diagrammatic representation of a machine in the example form of a computing device 1100 within which a set of instructions, for causing the machine to perform any one or more of the methodologies discussed herein, may be executed. In alternative embodiments, the machine may be connected (e.g., networked) to other machines in a Local Area Network (LAN), an intranet, an extranet, or the Internet. The computing device 1100 may correspond, for example, to computing device 105 and/or computing device 106 of **FIG. 1****.** The machine may operate in the capacity of a server or a client machine in a client-server network environment, or as a peer machine in a peer-to-peer (or distributed) network environment. The machine may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single machine is illustrated, the term "machine" shall also be taken to include any collection of machines (e.g., computers) that individually or jointly execute a set (or multiple sets) of instructions to perform any one or more of the methodologies discussed herein.

The example computing device 1100 includes a processing device 1102, a main memory 1104 (e.g., read-only memory (ROM), flash memory, dynamic random access memory (DRAM) such as synchronous DRAM (SDRAM), etc.), a static memory 1106 (e.g., flash memory, static random access memory (SRAM), etc.), and a secondary memory (e.g., a data storage device 1128), which communicate with each other via a bus 1108.

Processing device 1102 represents one or more general-purpose processors such as a microprocessor, central processing unit, or the like. More particularly, the processing device 1102 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 1102 may also be one or more special-purpose processing devices such as an application specific integrated circuit (ASIC), a field programmable gate array (FPGA), a digital signal processor (DSP), network processor, or the like. Processing device 1102 is configured to execute the processing logic (instructions 1126) for performing operations and steps discussed herein.

The computing device 1100 may further include a network interface device 1122 for communicating with a network 1164. The computing device 1100 also may include a video display unit 1110 (e.g., a liquid crystal display (LCD) or a cathode ray tube (CRT)), an alphanumeric input device 1112 (e.g., a keyboard), a cursor control device 1114 (e.g., a mouse), and a signal generation device 1120 (e.g., a speaker).

The data storage device 1128 may include a machine-readable storage medium (or more specifically a non-transitory computer-readable storage medium) 1124 on which is stored one or more sets of instructions 1126 embodying any one or more of the methodologies or functions described herein, such as instructions for intraoral scan application 1115, which may correspond to intraoral scan application 115 of **FIG. 1****.** A non-transitory storage medium refers to a storage medium other than a carrier wave. The instructions 1126 may also reside, completely or at least partially, within the main memory 1104 and/or within the processing device 1102 during execution thereof by the computing device 1100, the main memory 1104 and the processing device 1102 also constituting computer-readable storage media.

The computer-readable storage medium 1124 may also be used to store dental modeling logic 1150, which may include one or more machine learning modules, and which may perform the operations described herein above. The computer readable storage medium 1124 may also store a software library containing methods for the intraoral scan application 115. While the computer-readable storage medium 1124 is shown in an example embodiment to be a single medium, the term "computer-readable storage medium" should be taken to include a single medium or multiple media (e.g., a centralized or distributed database, and/or associated caches and servers) that store the one or more sets of instructions. The term "computer-readable storage medium" shall also be taken to include any medium other than a carrier wave that is capable of storing or encoding a set of instructions for execution by the machine and that cause the machine to perform any one or more of the methodologies of the present disclosure. The term "computer-readable storage medium" shall accordingly be taken to include, but not be limited to, solid-state memories, and optical and magnetic media.

It is to be understood that the above description is intended to be illustrative, and not restrictive. Many other embodiments will be apparent upon reading and understanding the above description. Although embodiments of the present disclosure have been described with reference to specific example embodiments, it will be recognized that the disclosure is not limited to the embodiments described, but can be practiced with modification and alteration within the scope of the appended claims. Accordingly, the specification and drawings are to be regarded in an illustrative sense rather than a restrictive sense. The scope of the disclosure should, therefore, be determined with reference to the appended claims, along with the full scope to which such claims are entitled.

## Claims

1. An intraoral scanning system, comprising:
an intraoral scanner (20) comprising a plurality of cameras (24) configured to generate a first set of intraoral images, each intraoral image from the first set of intraoral images being associated with a respective camera of the plurality of cameras; and
a computing device (105) configured to:
receive (502, 602) the first set of intraoral images;
determine (505, 605) that a first intraoral image of the first set of intraoral images that is associated with a first camera of the plurality of cameras satisfies one or more criteria;
select (510) the first camera of the plurality of cameras that is associated with the first intraoral image of the first set of intraoral images that satisfies the one or more criteria, wherein selecting the first camera comprises one of:
outputting (610) a recommendation for selection of the first camera and receiving user input to select the first camera; or
automatically selecting the first camera without user input; and
output (515, 618) the first intraoral image associated with the first camera to a display.

2. The intraoral scanning system of claim 1, wherein the plurality of cameras (24) comprises an array of cameras, each camera in the array of cameras having a unique position and orientation in the intraoral scanner (20) relative to other cameras in the array of cameras, and wherein the first set of intraoral images comprises at least one of near infrared (NIR) images or color images.

3. The intraoral scanning system of claim 1, wherein the first set of intraoral images is to be generated at a first time during intraoral scanning, and wherein the computing device (105) is further configured to:
receive a second set of intraoral images generated by the intraoral scanner (20) at a second time;
select a second camera of the plurality of cameras (24) that is associated with a second intraoral image of the second set of intraoral images that satisfies the one or more criteria; and
output the second intraoral image associated with the second camera to the display.

4. The intraoral scanning system of claim 1, wherein the computing device (105) is further configured to:
determine, for each intraoral image of the first set of intraoral images, a tooth area depicted in the intraoral image; and
select the first camera responsive to determining that the first intraoral image associated with the first camera has a largest tooth area as compared to a remainder of the first set of intraoral images.

5. The intraoral scanning system of claim 4, wherein the computing device (105) is further configured to perform the following for each intraoral image of the first set of intraoral images:
input the intraoral image into a trained machine learning model that performs classification of the intraoral image to identify teeth in the intraoral image, wherein the tooth area for the intraoral image is based on a result of the classification, in particular wherein the classification comprises pixel-level classification or patch-level classification, and wherein the tooth area for the intraoral image is determined based on a number of pixels classified as teeth.

6. The intraoral scanning system of claim 1, wherein the computing device (105) is further configured to:
determine, for each intraoral image of the first set of intraoral images, a score based at least in part on a number of pixels in the intraoral image classified as teeth, wherein the one or more criteria comprise one or more scoring criteria.

7. The intraoral scanning system of claim 6, wherein the computing device (105) is further configured to:
adjust scores for one or more intraoral images of the first set of intraoral images based on scores of one or more other intraoral images of the first set of intraoral images, in particular wherein the one or more scores are adjusted using a weighting matrix.

8. The intraoral scanning system of claim 7, wherein the computing device (105) is further configured to:
determine an area of an oral cavity being scanned based on processing of the first set of intraoral images; and
select the weighting matrix based on the area of the oral cavity being scanned.

9. The intraoral scanning system of claim 8, wherein the computing device (105) is further configured to:
input the first set of intraoral images into a trained machine learning model, wherein the trained machine learning model outputs an indication of the area of the oral cavity being scanned.

10. The intraoral scanning system of claim 8, wherein the area of the oral cavity being scanned comprises one of an upper dental arch, a lower dental arch, or a bite.

11. The intraoral scanning system of claim 8, wherein the computing device (105) is further configured to:
determine, for each intraoral image of the first set of intraoral images, a restorative object area depicted in the intraoral image; and
select the first camera responsive to determining that the first intraoral image associated with the first camera has a largest restorative object area as compared to a remainder of the first set of intraoral images.

12. The intraoral scanning system of claim 8, wherein the computing device (105) is further configured to:
determine, for each intraoral image of the first set of intraoral images, a margin line area depicted in the intraoral image; and
select the first camera responsive to determining that the first intraoral image associated with the first camera has a largest margin line area as compared to a remainder of the first set of intraoral images.

13. The intraoral scanning system of claim 1, wherein the computing device (105) is further configured to:
select a second camera of the plurality of cameras (24) that is associated with a second intraoral image of the first set of intraoral images that satisfies the one or more criteria;
generate a combined image based on the first intraoral image and the second intraoral image; and
output the combined image to the display.

14. The intraoral scanning system of claim 1, wherein the computing device (105) is further configured to:
output a remainder of the first set of intraoral images to the display, wherein the first intraoral image is emphasized on the display.

15. The intraoral scanning system of claim 1, wherein the computing device (105) is further configured to:
determine a score for each image of the first set of intraoral images;
determine that the first intraoral image associated with the first camera has a highest score;
determine the score for a second intraoral image of the first set of intraoral images associated with a second camera that was selected for a previous set of intraoral images;
determine a difference between the score for the first intraoral image and the score for the second intraoral image; and
select the first camera associated with the first intraoral image responsive to determining that the difference exceeds a difference threshold.

## Patentansprüche

1. System für intraorale Abtastung, umfassend:
einen Intraoralscanner (20), der eine Vielzahl von Kameras (24) umfasst, die dazu konfiguriert sind, einen ersten Satz von intraoralen Bildern zu erzeugen, wobei jedes intraorale Bild aus dem ersten Satz von intraoralen Bildern einer jeweiligen Kamera der Vielzahl von Kameras zugeordnet ist; und
eine Rechenvorrichtung (105), die zu Folgendem konfiguriert ist:
Empfangen (502, 602) des ersten Satzes von intraoralen Bildern;
Bestimmen (505, 605), dass ein erstes intraorales Bild des ersten Satzes von intraoralen Bildern, das einer ersten Kamera der Vielzahl von Kameras zugeordnet ist, ein oder mehrere Kriterien erfüllt;
Auswählen (510) der ersten Kamera der Vielzahl von Kameras, die dem ersten intraoralen Bild des ersten Satzes von intraoralen Bildern zugeordnet ist, das das eine oder die mehreren Kriterien erfüllt, wobei das Auswählen der ersten Kamera eines von Folgendem umfasst:
Ausgeben (610) einer Empfehlung zur Auswahl der ersten Kamera und Empfangen einer Benutzereingabe, um die erste Kamera auszuwählen; oder
automatisches Auswählen der ersten Kamera ohne Benutzereingabe; und Ausgeben (515, 618) des ersten intraoralen Bildes, das der ersten Kamera zugeordnet ist, an eine Anzeige.

2. System für intraorale Abtastung nach Anspruch 1, wobei die Vielzahl von Kameras (24) ein Array von Kameras umfasst, wobei jede Kamera in dem Array von Kameras eine einzigartige Position und Ausrichtung in dem Intraoralscanner (20) relativ zu anderen Kameras in dem Array von Kameras aufweist, und wobei der erste Satz von intraoralen Bildern mindestens eines von Nahinfrarot- (NIR-) Bildern oder Farbbildern umfasst.

3. System für intraorale Abtastung nach Anspruch 1, wobei der erste Satz von intraoralen Bildern zu einem ersten Zeitpunkt während des intraoralen Abtastens zu erzeugen ist und wobei die Rechenvorrichtung (105) ferner zu Folgendem konfiguriert ist:
Empfangen eines zweiten Satzes von intraoralen Bildern, die von dem Intraoralscanner (20) zu einem zweiten Zeitpunkt erzeugt werden;
Auswählen einer zweiten Kamera der Vielzahl von Kameras (24), die einem zweiten intraoralen Bild des zweiten Satzes von intraoralen Bildern zugeordnet ist, das das eine oder die mehreren Kriterien erfüllt; und Ausgeben des zweiten intraoralen Bildes, das der zweiten Kamera zugeordnet ist, an die Anzeige.

4. System für intraorale Abtastung nach Anspruch 1, wobei die Rechenvorrichtung (105) ferner zu Folgendem konfiguriert ist:
Bestimmen, für jedes intraorale Bild des ersten Satzes von intraoralen Bildern, eines Zahnbereichs, der in dem intraoralen Bild dargestellt ist; und
Auswählen der ersten Kamera als Reaktion auf das Bestimmen, dass das erste intraorale Bild, das der ersten Kamera zugeordnet ist, einen größten Zahnbereich im Vergleich zu einem Rest des ersten Satzes von intraoralen Bildern aufweist.

5. System für intraorale Abtastung nach Anspruch 4, wobei die Rechenvorrichtung (105) ferner dazu konfiguriert ist, für jedes intraorale Bild des ersten Satzes von intraoralen Bildern Folgendes durchzuführen: Eingeben des intraoralen Bildes in ein trainiertes Modell für maschinelles Lernen, das eine Klassifizierung des intraoralen Bildes durchführt, um Zähne in dem intraoralen Bild zu identifizieren, wobei der Zahnbereich für das intraorale Bild auf einem Ergebnis der Klassifizierung basiert, insbesondere wobei die Klassifizierung eine Klassifizierung auf Pixelebene oder eine Klassifizierung auf Patch-Ebene umfasst, und wobei der Zahnbereich für das intraorale Bild basierend auf einer Anzahl von Pixeln bestimmt wird, die als Zähne klassifiziert sind.

6. System für intraorale Abtastung nach Anspruch 1, wobei die Rechenvorrichtung (105) ferner zu Folgendem konfiguriert ist:
Bestimmen, für jedes intraorale Bild des ersten Satzes von intraoralen Bildern, einer Punktezahl basierend zumindest teilweise auf einer Anzahl von Pixeln in dem intraoralen Bild, die als Zähne klassifiziert sind, wobei das eine oder die mehreren Kriterien ein oder mehrere Bewertungskriterien umfassen.

7. System für intraorale Abtastung nach Anspruch 6, wobei die Rechenvorrichtung (105) ferner zu Folgendem konfiguriert ist:
Einstellen von Punktezahlen für ein oder mehrere intraorale Bilder des ersten Satzes von intraoralen Bildern basierend auf Punktezahlen eines oder mehrerer anderer intraoraler Bilder des ersten Satzes von intraoralen Bildern, insbesondere wobei die eine oder mehreren Punktezahlen unter Verwendung einer Gewichtungsmatrix eingestellt werden.

8. System für intraorale Abtastung nach Anspruch 7, wobei die Rechenvorrichtung (105) ferner zu Folgendem konfiguriert ist:
Bestimmen eines Bereichs einer Mundhöhle, der abgetastet wird, basierend auf der Verarbeitung des ersten Satzes von intraoralen Bildern; und
Auswählen der Gewichtungsmatrix basierend auf dem Bereich der Mundhöhle, der abgetastet wird.

9. System für intraorale Abtastung nach Anspruch 8, wobei die Rechenvorrichtung (105) ferner zu Folgendem konfiguriert ist:
Eingeben des ersten Satzes von intraoralen Bildern in ein trainiertes Modell für maschinelles Lernen, wobei das trainierte Modell für maschinelles Lernen eine Angabe des Bereichs der Mundhöhle ausgibt, der abgetastet wird.

10. System für intraorale Abtastung nach Anspruch 8, wobei der Bereich des oralen Hohlraums, der abgetastet wird, eines von einem oberen Zahnbogen, einem unteren Zahnbogen oder einem Biss umfasst.

11. System für intraorale Abtastung nach Anspruch 8, wobei die Rechenvorrichtung (105) ferner zu Folgendem konfiguriert ist:
Bestimmen, für jedes intraorale Bild des ersten Satzes von intraoralen Bildern, eines restaurativen Objektbereichs, der in dem intraoralen Bild dargestellt ist; und
Auswählen der ersten Kamera als Reaktion auf das Bestimmen, dass das erste intraorale Bild, das der ersten Kamera zugeordnet ist, einen größten restaurativen Objektbereich im Vergleich zu einem Rest des ersten Satzes von intraoralen Bildern aufweist.

12. System für intraorale Abtastung nach Anspruch 8, wobei die Rechenvorrichtung (105) ferner zu Folgendem konfiguriert ist:
Bestimmen, für jedes intraorale Bild des ersten Satzes von intraoralen Bildern, eines Randlinienbereichs, der in dem intraoralen Bild dargestellt ist; und
Auswählen der ersten Kamera als Reaktion auf das Bestimmen, dass das erste intraorale Bild, das der ersten Kamera zugeordnet ist, einen größten Randlinienbereich im Vergleich zu einem Rest des ersten Satzes von intraoralen Bildern aufweist.

13. System für intraorale Abtastung nach Anspruch 1, wobei die Rechenvorrichtung (105) ferner zu Folgendem konfiguriert ist:
Auswählen einer zweiten Kamera der Vielzahl von Kameras (24), die einem zweiten intraoralen Bild des ersten Satzes von intraoralen Bildern zugeordnet ist, das das eine oder die mehreren Kriterien erfüllt;
Erzeugen eines kombinierten Bildes basierend auf dem ersten intraoralen Bild und dem zweiten intraoralen Bild; und
Ausgeben des kombinierten Bildes an die Anzeige.

14. System für intraorale Abtastung nach Anspruch 1, wobei die Rechenvorrichtung (105) ferner zu Folgendem konfiguriert ist:
Ausgeben eines Restes des ersten Satzes von intraoralen Bildern an die Anzeige, wobei das erste intraorale Bild auf der Anzeige hervorgehoben wird.

15. System für intraorale Abtastung nach Anspruch 1, wobei die Rechenvorrichtung (105) ferner zu Folgendem konfiguriert ist:
Bestimmen einer Punktezahl für jedes Bild des ersten Satzes von intraoralen Bildern;
Bestimmen, dass das erste intraorale Bild, das der ersten Kamera zugeordnet ist, eine höchste Punktezahl aufweist;
Bestimmen der Punktezahl für ein zweites intraorales Bild des ersten Satzes von intraoralen Bildern, der einer zweiten Kamera zugeordnet ist, die für einen vorherigen Satz von intraoralen Bildern ausgewählt wurde;
Bestimmen einer Differenz zwischen der Punktezahl für das erste intraorale Bild und der Punktezahl für das zweite intraorale Bild; und
Auswählen der ersten Kamera, die dem ersten intraoralen Bild zugeordnet ist, als Reaktion auf das Bestimmen, dass die Differenz einen Differenzschwellenwert überschreitet.

## Revendications

1. Système pour balayage intra-buccal comprenant :
un scanner intra-buccal (20) comprenant une pluralité de caméras (24) configurées pour générer un premier ensemble d'images intra-buccales, chaque image intra-buccale du premier ensemble d'images intra-buccales étant associée à une caméra respective de la pluralité de caméras ; et
un dispositif informatique (105) configuré pour :
recevoir (502, 602) le premier ensemble d'images intra-buccales ;
déterminer (505, 605) qu'une première image intra-buccale du premier ensemble d'images intra-buccales qui est associée à une première caméra de la pluralité de caméras satisfait un ou plusieurs critères ;
sélectionner (510) la première caméra de la pluralité de caméras qui est associée à la première image intra-buccale du premier ensemble d'images intra-buccales qui satisfait auxdits un ou plusieurs critères, ladite sélection de la première caméra comprenant une opération parmi :
l'émission (610) d'une recommandation pour la sélection de la première caméra et la réception de l'entrée utilisateur pour sélectionner la première caméra ; ou
la sélection automatique de la première caméra sans entrée utilisateur ; et
délivrer (515, 618) la première image intra-buccale associée à la première caméra à un dispositif d'affichage.

2. Système pour balayage intra-buccal selon la revendication 1, ladite pluralité de caméras (24) comprenant un réseau de caméras, chaque caméra du réseau de caméras présentant une position et une orientation uniques dans le scanner intra-buccal (20) par rapport aux autres caméras du réseau de caméras, et ledit premier ensemble d'images intra-buccales comprenant au moins une image parmi les images dans le proche infrarouge (NIR) ou les images en couleurs.

3. Système pour balayage intra-buccal selon la revendication 1, ledit premier ensemble d'images intra-buccales devant être généré à un premier moment pendant le balayage intra-buccal, et ledit dispositif informatique (105) étant en outre configuré pour :
recevoir un second ensemble d'images intra-buccales générées par le scanner intra-buccal (20) à un second moment ;
sélectionner une seconde caméra de la pluralité de caméras (24) qui est associée à une seconde image intra-buccale du second ensemble d'images intra-buccales qui satisfait auxdits un ou plusieurs critères ; et
délivrer la seconde image intra-buccale associée à la seconde caméra au dispositif d'affichage.

4. Système pour balayage intra-buccal selon la revendication 1, ledit dispositif informatique (105) étant en outre configuré pour :
déterminer, pour chaque image intra-buccale du premier ensemble d'images intra-buccales, une zone dentaire représentée dans l'image intra-buccale ; et
sélectionner la première caméra en réponse à la détermination que la première image intra-buccale associée à la première caméra comporte une zone dentaire plus grande par rapport au reste du premier ensemble d'images intra-buccales.

5. Système pour balayage intra-buccal selon la revendication 4, ledit dispositif informatique (105) étant en outre configuré pour effectuer les opérations suivantes pour chaque image intra-buccale du premier ensemble d'images intra-buccales :
entrer l'image intra-buccale dans un modèle d'apprentissage automatique entraîné qui effectue une classification de l'image intra-buccale pour identifier les dents dans l'image intra-buccale, ladite zone dentaire pour l'image intra-buccale étant basée sur un résultat de la classification, en particulier ladite classification comprenant une classification au niveau des pixels ou une classification au niveau des patchs, et ladite zone dentaire pour l'image intra-buccale étant déterminée sur la base d'un nombre de pixels classés en tant que dents.

6. Système pour balayage intra-buccal selon la revendication 1, ledit dispositif informatique (105) étant en outre configuré pour :
déterminer, pour chaque image intra-buccale du premier ensemble d'images intra-buccales, un score basé au moins en partie sur un nombre de pixels dans l'image intra-buccale classés comme dents, lesdits un ou plusieurs critères comprenant un ou plusieurs critères de score.

7. Système pour balayage intra-buccal selon la revendication 6, ledit dispositif informatique (105) étant en outre configuré pour :
ajuster les scores pour une ou plusieurs images intra-buccales du premier ensemble d'images intra-buccales sur la base des scores d'une ou plusieurs autres images intra-buccales du premier ensemble d'images intra-buccales, en particulier lesdits scores étant ajustés à l'aide d'une matrice de pondération.

8. Système pour balayage intra-buccal selon la revendication 7, ledit dispositif informatique (105) étant en outre configuré pour :
déterminer une zone d'une cavité buccale en cours de balayage sur la base du traitement du premier ensemble d'images intra-buccales ; et
sélectionner la matrice de pondération sur la base de la zone de la cavité buccale en cours de balayage.

9. Système pour balayage intra-buccal selon la revendication 8, ledit dispositif informatique (105) étant en outre configuré pour :
entrer le premier ensemble d'images intra-buccales dans un modèle d'apprentissage automatique entraîné, ledit modèle d'apprentissage automatique entraîné produisant une indication de la zone de la cavité buccale en cours de balayage.

10. Système de balayage intra-buccal selon la revendication 8, ladite zone de la cavité buccale en cours de balayage comprenant un élément parmi un arc dentaire supérieur, un arc dentaire inférieur ou une morsure.

11. Système pour balayage intra-buccal selon la revendication 8, ledit dispositif informatique (105) étant en outre configuré pour :
déterminer, pour chaque image intra-buccale du premier ensemble d'images intra-buccales, une zone d'objet de restauration représentée dans l'image intra-buccale ; et
sélectionner la première caméra en réponse à la détermination que la première image intra-buccale associée à la première caméra comporte une zone d'objet de restauration plus grande par rapport au reste du premier ensemble d'images intra-buccales.

12. Système pour balayage intra-buccal selon la revendication 8, ledit dispositif informatique (105) étant en outre configuré pour :
déterminer, pour chaque image intra-buccale du premier ensemble d'images intra-buccales, une zone de ligne de marge représentée dans l'image intra-buccale ; et
sélectionner la première caméra en réponse à la détermination que la première image intra-buccale associée à la première caméra comporte une zone de ligne de marge plus grande par rapport au reste du premier ensemble d'images intra-buccales.

13. Système pour balayage intra-buccal selon la revendication 1, ledit dispositif informatique (105) étant en outre configuré pour :
sélectionner une seconde caméra de la pluralité de caméras (24) qui est associée à une seconde image intra-buccale du premier ensemble d'images intra-buccales qui satisfait auxdits un ou plusieurs critères ;
générer une image combinée sur la base de la première image intra-buccale et de la seconde image intra-buccale ; et
délivrer l'image combinée au dispositif d'affichage.

14. Système pour balayage intra-buccal selon la revendication 1, ledit dispositif informatique (105) étant en outre configuré pour :
délivrer le reste du premier ensemble d'images intra-buccales au dispositif d'affichage, ladite première image intra-buccale étant mise en évidence sur le dispositif d'affichage.

15. Système pour balayage intra-buccal selon la revendication 1, ledit dispositif informatique (105) étant en outre configuré pour :
déterminer un score pour chaque image du premier ensemble d'images intra-buccales ;
déterminer que la première image intra-buccale associée à la première caméra présente un score plus élevé ;
déterminer le score pour une seconde image intra-buccale du premier ensemble d'images intra-buccales associées à une seconde caméra qui a été sélectionnée pour un ensemble précédent d'images intra-buccales ;
déterminer la différence entre le score pour la première image intra-buccale et le score pour la seconde image intra-buccale ; et
sélectionner la première caméra associée à la première image intra-buccale en réponse à la détermination que la différence dépasse un seuil de différence.
